(19) Europäisches Patentamt
European Patent Office
Office européen des brevets

(11) EP 1 300 467 A1

(12) **EUROPEAN PATENT APPLICATION**
published in accordance with Art. 158(3) EPC

(43) Date of publication:
**09.04.2003 Bulletin 2003/15**

(21) Application number: **01947952.6**

(22) Date of filing: **10.07.2001**

(51) Int Cl.[7]: **C12N 15/12**, C12P 21/02, C07K 14/47, C07K 16/18, C12N 1/15, C12N 1/19, C12N 1/21, C12N 5/00, A61K 38/17, A61K 31/7088, G01N 33/68, A61P 25/04

(86) International application number:
**PCT/JP01/05970**

(87) International publication number:
**WO 02/004642 (17.01.2002 Gazette 2002/03)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **12.07.2000 JP 2000215886**
**24.07.2000 JP 2000227553**

(71) Applicant: **Takeda Chemical Industries, Ltd.**
**Osaka-shi, Osaka 541-0045 (JP)**

(72) Inventors:
- **HINUMA, Shuji**
**Tsukuba-shi, Ibaraki 305-0821 (JP)**
- **FUKUSUMI, Shoji**
**Tsukuba-shi, Ibaraki 305-0044 (JP)**

(74) Representative: **Lewin, John Harvey**
**Takeda Euro IP Department,**
**11-12 Charles II Street**
**London SW1Y 4QU (GB)**

(54) **NOVEL POLYPEPTIDE AND DNA THEREOF**

(57) The present invention relates to a novel polypeptide or a salt thereof having an analgesic peptide-like structure. The protein (polypeptide) of the present invention, a partial peptide thereof, and a DNA encoding the same can be used for preparation of an antibody and an antiserum to the protein, construction of the expression system for the protein (polypeptide), screening of a pharmaceutical candidate compound using the expression system, etc.

EP 1 300 467 A1

[Sequence Listing]

<110> Takeda Chemical Industries, Ltd.

<120> Novel Polypeptide and its DNA

<130> P2001-171PCT

<150> JP 2000-215886
<151> 2000-07-12
<150> JP 2000-227553
<151> 2000-07-24

<160> 5

<210> 1
<211> 73
<212> PRT
<213> Human

<400> 1
Met Arg His Ser Gln Glu Ala Leu Val Phe Ala Leu Thr Pro Leu Leu
                5                   10                  15
Leu Ala Asp Pro Gly Glu Ala Ile His Ala Glu Leu Gln Lys Gly His
            20                  25                  30
Tyr Ser Leu Cys Ala Ala Leu Thr Arg Arg Ile Arg Glu Leu Leu Gln
        35                  40                  45
Arg Tyr Gly Gly Phe Leu Glu Glu Leu Thr Glu His Ala Gly Val Thr
    50                  55                  60
Pro Gln Ser Ala Leu Lys Ser Phe Lys
65                  70          73

<210> 2
<211> 219
<212> DNA
<213> Human

<400> 2
ATGCGTCACA GCCAGGAAGC CCTGGTTTTC GCTCTGACCC CACTGCTTCT GGCTGACCCT 60
GGAGAAGCCA TCCACGCTGA GCTGCAGAAG GGCCACTACA GCCTGTGTGC AGCCCTCACA 120
AGGCGTATAC GGGAATTACT GCAGCGATAC GGGGGATTCC TCGAGGAGCT CACTGAGCAT 180
GCAGGTGTTA CACCCCAGAG CGCGCTGAAA TCATTCAAA 219

<210> 3
<211> 27
<212> DNA
<213> Artificial Sequence

<220>
<223> Primer

<400> 3
CTCAGTGGCT GAAGGCACAT GCGTCAC 27

<210> 4
<211> 27
<212> DNA
<213> Artificial Sequence

<220>
<223> Primer

<400> 4
GGGAGGCTTA GGCTTGGCTC ATTTGAA 27

<210> 5

<211> 39265
<212> DNA
<213> Human

<400> 5
gatccacccg ccttgacttc ccaaagtgct gggattacag gcgtgagcca ctgagcccag
60ctgtaaggtt cttatactag atgaaggtag actgtgataa attaaagaca tactataaac
120
cctaaagcac ctgttaaagt caacaaacaa acaagaaaac aaaagactgt cagtgaataa 1
80
gacaacaaat aaactgaaaa gattgtttta aaaactaaat tcaaaagaag accagtaaga 2
40
gggaaagggg cctggcgcag tggctcacgc ctgtaatccc aacactttgg gaggctgagg 3
00
tgggcagatc actttaggtc aggagttcaa gaccagcctc accaagatgg tgaaaccctg 3
60
tctctaccaa aaatacaaaa attatccagg catcgtggca tgcacctgta gtcccagcca 4
20
ctcaggaggc tgaggcagga gaatcactta agcctgggag gcggaggttg cagtgagccg 4
80
agactgtgcc actgcactcc agcctgggcg acagagtgag actctgtctc aaaaagaaaa 5
40
gaaagaaaaa aaaatccatg gcagacagag gccctgcagt gggatcgggg tgaaagtcca 6
00
gcatctgtga cctcagacgt gtcacatcct tgctcatcac agcttcagca tcgatcacac 6
60
tggcattcat tcattcaaga gacatttgct ggctgggcgc agaggctcac acctgtaatc 7
20
ccagcacttt gggaggccaa gaccagtgga tcacctgaag tcaggagttc aagaccagcc 7
80
tgcccaacat ggtgaaaccc tgtctctact aaaaatacaa aaatgttagc tgggcgtggt 8
40
gtcaggcacc tgtaatccca gctactcggg aggctgaggc aggagaatca cttgatcctg 9
00
ggaggtggag gttgcagtga gccaagatag caccattgca ctccagcctg ggcaacgaga 9
60

gcgaaactct gtcttaaaaa aaaaaaagag agagagagaa ttgctgaacg cccactaagt 10
20
gccaggcacc attctggttg cggcctcatt gctgaataat gcgggcagaa accccaactc 10
80
cgtggagtgc ctgcttggtg agaagactcc cttgtagaac tctgtgaggg tcagagacga 11
40
tggatgggag gtcctgccag gaggaggcgc tcaggaagtt tcttcgctgc tcctccccac 12
00
tccttcactc ctttcttgtt gctcctcttt caggcctggg tgtttgggga gcttcattct 12
60
ctcattccac agatatgcct gcagtgcccc gcctggcctg tgctgagcct ggtccaggtt 13
20
tgttgaatgc agacgtgctc agagaggtca ttccggctgc tggtcactgg gaactccgtt 13
80
gcatctgcct cgggcccagg tgcccccctt tcactggagc atcagcgaag agcagtcacc 14
40
cccctccccg ccctgggtat ggcaggccca ggctgcagag ctttcttcgt gaaacaggag 15
00
ctaaacatga tcaagatctt gccacgtccc aggcaggagt ctcaccacac tgctggtgtc 15
60
atctcatgag actgcacaac aaaccttcgc agcaggtccc cgagtgtacc ctttcacagc 16
20
tatggacagg acccaaaagt ctgagtcgag cgtgtgtgtc actaccctgt atgtggattc 16
80
acagcctctg gctatcttag aaggtccttc ccccatggcc tggctctgcc cgtgaaccgt 17
40
tgaatgtctc tgctcccttt gccacttccg ccttcctgat actggggaac caggtcatct 18
00
ctctactctt tcctggaatc ttctcttttc caaggatcat ggtgactgag aaactgagga 18
60
gctgcacagg cccaagacaa gccctctccc accagcccac aaatgtactg cccatgggcc 19
20
gggcatggtg gctcatgcct ataatcccag cactttggga ggccgaggcg ggcggatcac 19
80
ctgaggtcag gagttcgaga ccagcctggc caacatggtg aaaccccatc tctactaaaa 20
40

atataaaaat tagccgggtg tggtggtgtg cacctgtaat agccactcgg gaggctgagg 2100
caggagcatc gcttgcacct gggagacaga ggttgcagtg agctgagatc gcaccactgc 2160
actccattct gggtgacaga acaagagtcc atttcaaaaa aataaaaaat gtactgtcca 2220
tgtttactga tgactttatg tagttagtgg atcctgccag cacagccaca gttcctgctg 2280
atctctgtcc cgcagttgac caccccgtct gtgtttttgc cccagcctgt attgctgggc 2340
ttgagtcttc tgtcctcacg accctcaccg gggactcaga cacagccctt ctgctctggg 2400
gacatgtcag tccttggcct gagcgtccag gctctgcgcg gccccgttgt ccttacaggg 2460
actccgtccc tggggaaact tcccacgtgt cagaaccttc ccttcctttg tttgagaaaa 2520
caaaaaattc ttcctctctt gaccaggagc agtggctcac gctggtaatc ccagcacttt 2580
gggaggccga gacaggagaa ttgcttgagg ccaggagttc aagaccaaca tagcaagatc 2640
ccatctgttt atagatctat atctatgtat catctctcta tatagtacat gtaatatgta 2700
tataatatat aataatcata tatataattc ttcctttttt gcggggggag ggggttggag 2760
tctcactctg tcacccaggc tggagtgcag tggcatgatc taggctcact gcaacctccc 2820
cgtcctggtt caagcgattc tcctgtctca gcctcccaag tagctggaac tacaggcatg 2880
tgccaccgca tctggctaat ttttgtatct tttttttttt tttttttttt gagatggagt 2940
ttcactctgt cgcccaggct ggagtgcaat ggtgggatct cagctcactg caacttccgc 3000
ctccctggtt caagtgattc tcctgcctca gtctcccaag cagcagggat taggcgcctg 3060
ccaccacacc tggctaattt tttgtatttt cagtagagat ggggtttcac catgttagcc 3120

aggctggtct tgaactcctg acctcaggtg atccgcccgc ctcggcctcc ccaagtgttg 3180
ggattacagg cgtgagccac tgtgcctggc caattcttcc tttcttgaat gcccttttct 3240
gccatcctaa ttttcccatc gttcagggtc aaggttgatg ctcaccttcc ccagtaagcc 3300
ctccctgatg tcttcccaat tcctgtgggg taagcagggc agctcagtgg ctgaaggcac 3360
atgcgtcaca gccaggaagc cctggttttc gctctgaccc cactgcttct ggctgaccct 3420
ggagaagcca tccacgctga gctgcagaag ggccactaca gcctgtgtgc agccctcaca 3480
aggcgtatac gggaattact gcagcgatac gggggattcc tcgaggagct cactgagcat 3540
gcaggtgtta caccccagag cgcgctgaaa tcattcaaat gagccaagcc taagcctccc 3600
taccgcctcg cccacccccct cccatggaaa ccacattacg ggcttcaccc acagttctgc 3660
cctctcctgc ctcctgaccg actccagcac ttccccgtgt ggccctgtgt ggcttggggt 3720
acccttctc ctggaaactg tcgtaaacta tcctttcaat ggcaattatc tcctgatctg 3780
gtggccttac tgaacctcaa attttcaata aatacgttgt tttttaagcc aggcttaata 3840
gcatgcactt gtggtctcag ctacttggga ggctgaggtg ggaggatcgc ttgaggccag 3900
gagtttgtgg ccgcagtgca gtataattgc acctgtgaag agccactgca ctccagcctg 3960
ggcaacgcag ggagaccctg tgtccaaaaa ataattatta tattttaaag acaagcagac 4020
tatgcatgga gtcccgaggg tgaacaggtg tcgggggca gcgggagcgt ggagaaagcc 4080
gggaggaggt gggaacagga aaccctcagg gtcacacccc agaaggagaa ggccctagcc 4140
tgaatgggac tgctgggaag ggctgaaca ccagcaggga gtggagctgc ggaaacacac 4200

acggaagtgg ggcatgggtg ctgggtggac actccacttc cagataagag gggatcttgt 4260
ggatggtggt ggaggcgctc ctggccctac tgggctccgg cactcagcct gccctgccac 4320
agaggcagcc catactagag gctactgcac gtcaggagag gggcaggctc aaaaccagaa 4380
aagttatact ctgggctctg ccagccccac cctcccaatc caggaaaccc aattcatcca 4440
aatatgagta tcaatatcca cccaaagata ccagagggaa aaaagaaacc taaaaggagc 4500
gggtggccag tgcagtgctc actcctgaat cccagcactt tgggaggctg aggcgggagg 4560
atcgcttaag cccaggaggt caagactgca gtgagttgtg actatgtcac tgcactccag 4620
cccaggcaac aaagcaagac cctgtctcaa aaacaaacaa acaaaaaggg aacagcaaga 4680
cttccctgca gatgaagaaa acacagagaa aaccactgcc acaaagccaa gtgaaagtgg 4740
caagccagcg ggcagcccca tgatgcagct ggggaagtgg ccaggggagt ggctgcagcg 4800
gaaatggcca tggggaactc tgcggcccca ggaagaacag gtggacaggt gctggtggaa 4860
cttaggaagg actggaagca aaggccacag acacaagtct agagatgctg tggaaagtgc 4920
agccaggaag aaacagggac gtgaagaggc acaacagcag cagagcaagc aggcaggaga 4980
ggcggtggcg ggggaggcag cagcagggga agcagcggtg gggaggcagc agcaggcagg 5040
tccttggctg gcagggcaga gccaagacag gacagactcc aaggtagaag tccagacaac 5100
tttgctaaaa caaacgaatt gaggcggagc attactgctg agctttgcct cctgtcagat 5160
cagcggtggc attagatctt catagaagtg caaaccctac tgtgaactgc gcatgcgagg 5220
gatctaggtt gctcactcct aatgaaaatc taatgcctga tgatctgaag tggcacagtt 5280

tcatccccaa accatccctc ccaccacccc tactgtccat ggaaaaattg tcttccaaga 5340
aaccagtcac tggggcaaaa aatgttggtc aaaagtaccg ctgtactaga gtgaatttta 5400
gttaactgag agatgatcac agatagtaag aggaacaggg agtgagcact cacatattca 5460
actagaggac agggaccgag ataaaaacag cagcaagttg tgatgggtac gtacggtgtg 5520
ttccctgcca aatccacaca ctgatgtctt aaccccaagc accacagaac gtgaccttgt 5580
ttggaaatgg tgtcactgca aatgttatta gttaagatga ggtcactaag gtgggctcta 5640
atccaatgtg actggtgtcc ttattataag aagaggcaat caggacccac agacaggcac 5700
agggggaaga tgagaggatg ctgtttgcag gccaaggaca gcacccagga acagccttca 5760
ctcagggtcc tcagaagaaa ccaaccctgc tgatgtctgg actggacccc tgccctccag 5820
agctgtgaac aatgaattcc tctcacctaa gctgctctgc ggtgctttgt taatggcagc 5880
cctagcaaac tcatacacaa aggtaacaga atgtgtaaaa aaacaatttt acaaaggcag 5940
ctataacagt atgagacaag aaagcagaga gaggatggaa aatagaatcg ccctgctgat 6000
tccttcatat tatagttgga ttctaaagac atcacttaat gctgacaaac tgagaagttt 6060
agcatattaa agaggacaga ggcaaatact aagaaatata atagtattca ttcaaaaaat 6120
gggtgggccg ggcatggtgg ctcacacctg taatcccagc actttgggag gccgaggcag 6180
gtggatcacc tgaggtcagg agttcgagac cagcctggcc aacatggtga aatccatctc 6240
tactaaaaat acaaaaatta gccaggcttg gtggcgcgca tctgtaatcc cagctactag 6300
ggaggctgga gcaggagaat cgcctgaacc tgggaggtag aggttgcagt gagctgagat 6360

ggcgccattc cactccagcc tgggtgacag agcgaggcct gtctcaaaaa aaaaaaacaa 64
20
aaaaaaccat gggtggtaga aaagagagag aagggagaga ggggaaaggt ttacaaatta 64
80
tttcatagta gggaactatg agaactcttt taaaggttga ggggactaaa gatattattt 65
40
aagggtatga acagtaagta gcaactagac ccaaaaaaaa tcaagccttc ctaaaaacca 66
00
gaagagctac attttttaag agcaaatgca gattaagtaa tgactatatg tagtaaatgt 66
60
gaaataaaac aatttcaaaa agaacaaagt tggaagactc atgctacccg atttcaacac 67
20
ttactataga gctgcagtga ttaagacggt gtggcgctgg cgaaaggttc aggggaacag 67
80
aacacacagt ttagaaatag acctacacag atttatggcc aattgatttc agatgaaggt 68
40
acaagggcaa ttctacgaag acagtctttt cagcaactgg tcctggaaca ctggacatct 69
00
ttatgcaaaa acgaaccttc aaaatggtta tgccatttta tttatttatt tatttactat 69
60
tttttgagat ggagtcttgc tctgtcttcc aggctggagt gcagtggcga cagctcggct 70
20
cactgcaacc tccgtttccc aggttcaagt gattctcctg tctcagcctc ccaagtagct 70
80
gggattacag gcacctgcca ccacgcccgg ctaattttta tatttttagt agagatggat 71
40
ttcaccatct tggtcgggct ggtcttgaac tcctgagctc aagtgatcca cctgcctcgg 72
00
cctcccaaag tgctgggatt acaggagtga ggcaccacgc ccggccacaa gtcctttatt 72
60
atactgtatg ttttacaaat attttctccc actgtgagcc tttttatctg ttttcctaat 73
20
ttttaatttt tgatgaagtg caataattga cagaatgaat agatagaaaa ttggcaccag 73
80
gtgcagtggc tcacgcctgt aatcccagca ctttgggagg ccgaggaagg cagatcacct 74
40

gaggtcggga gttcgagacc agcctgacca acatggagaa accccatctc tattaaaaat 7500
acaaaattag ccgggcatgg tggcgcatgc ctgtaatctc agctactcag gaggctgaca 7560
ggagaactgc ttgaacccag gagacagagg ttgcagtgag ccaagaccgc gccattgcac 7620
tccagcctgg gcaacaagag caaaactctg tctcaagaaa aaaaaaagaa agaaagaaag 7680
aaaaaagaaa agaaaattgg caaggataca gaacttgaac tccactggac ctgacattaa 7740
aacacttgac tcaacaagag cagaaaacat tcttctcgag cacacacaga acatttacca 7800
ggagagacca cagtctgggc tatgaaacaa accctgttaa cttcaaaaag attcaagtgg 7860
tttttgttg ttttttgata cacggtctca ctctgttgcc caggctggag tgcagtggca 7920
ggaacatggc tcactgcagc ctcaacctcc tgggctcaag tgatcctccc acctcagctt 7980
tctgagtagc tgggactata ggcataggcg tgggcaaccg ccccagggtg attttttaaa 8040
tttttttgta gagacagggt ctcgctgcat tgcccaggct gacctcaatc tcctgggctc 8100
aaatgatcct cctgcctcag cctcccagta tcgggattac aggtgtgagc caccacgccc 8160
cagccaagga ctcaagttat acaaagtatg ttctctgacc acaatgaaat tcaactagaa 8220
atcagtaaca gaaagatctc tggtaaattc ccaaatattt ggaaactaaa tgacatacct 8280
ttaaataact cattagccaa agaataaatc aaagggaaat tagcaagtat tttgaactga 8340
atgaaaacac aacatatgaa aattcgtcag gtatcacaaa agcagactga actttttaga 8400
ccttacaaat gcatgactgt cccccttgcc tctgatcttc tgcctggggt ctgcctttcc 8460
ccaatctttg ttcactatac tgaatcctac atgacacagt caacctatga agaaatccag 8520

agatagactc tctaaaataa atggatttgg gaatagcagc ttatctggaa tactgcaacc 8580
gttgaggatg gtcttgcagt gagtcctgtt attggtctgt ttgtagaaat gtcttgtggt 8640
aagtcctgtt gcaggaatgt gtgcgtgagg gctgcttcat cacctccaac tgttttagtt 8700
tgacataagt gactccattt tggtaccggc aacgttcaca actttccatg tgacttcagc 8760
accctgcacc ccgatccttt ccctggccac tttgcaggac cactacctat gagactatgg 8820
gttccttgag agctagggct gggtctcatt cgctcctgaa actttcaccc agcagagtga 8880
tggcccacgc catgcatgac atgcgttgct caatgactgc atccactcag ccagtatgcc 8940
agtccccact gagcctcact ctcaacctcc ctggctggca taggttcctt gtgcagacat 9000
ccagtggcag taaaatattg ctgggttctc agcttccccc acctgcagcc ctcactggcc 9060
cacccagttg tcctgtctct gctcaggacg agaccccccc ccgcctcttc agtctctgta 9120
gccaggtgat ggcagtggcc ttttccagac cctcactggc cccttagttc actatgatga 9180
cttcacctct tgggaaccca tggggatgtc tttccctcca atacatctac atttgcctga 9240
cccagaatgt agtttcaaga tgggtccatg cctggctcca aatcccctta tcagagtaag 9300
gaaatacctt aattcctagt ttgctaaaaa ctcgttataa atggctgtcg cgtcatttac 9360
tgtgttcctg ttctgcaacc attgagatgg tcacgtgggt tttgctcctt taatctattg 9420
atatgaatta cactcttgat tctctggcat tgaaactgct ttgcaattct gaattaaatc 9480
cagtgtggca ctttgctaaa cttggtttat tattgtctaa aatcttgaca ttggtatttg 9540
tagggaccag ccccacaggg tccgtgggtc tctccctccc tgtgtgcagc aatgagagag 9600

tgtagaaata cacacacaag acaaagagat aaaagaaaag gcagctgggc ccgggagacc 9660
actactacca atgctcggag accggtagtg gccccgaatg tctggctgca ttgttattta 9720
ttggatacaa agcaaaaggg gcagggtaaa gagtgtgagt catctccaat gataggtaag 9780
gtcacgtgga tcacgtgtcc actggacagg gtgcccttcc ctgcctggca gccgaggcag 9840
agagagagag gagacagaga gaaagacagc ttatgccatt acttctgcat atcagagact 9900
tttagtactt tcactaattt actactgcta tctagaaggc agagccaggt gtacaggatg 9960
gaacatgaag gcggactagg agcgtgacca ctgaagcaca gcatcacagg gagacggtta 10020
ggcctccaga taactgcggg caggcctgcc ggatgtcagg ccctccacaa gaggtggaag 10080
agcagagtct tctctaaact cctccaggga aagggacact ccctttcccg gtctgctaag 10140
tagcgggtgt tgttccttga tactttttgc tactgctaga ccatggtcca cctggcaacg 10200
ggcgtcttcc cagacgctgg cgtcaccgct agaccaagga gccctctggt ggccctgtcc 10260
gggcataaca gaaggctcgc actcttgtct tctggtcaca cctatgtccc ctcagctcct 10320
atctctgtat ggcctggttt ttcctaggct atgattatag agcgaggatt attataatat 10380
tggaataaaa ggtaattgct acaaactaat gattaatgat attcatatgt aatcatatct 10440
aagatctata tctgctgtaa ctattcttgt tttatatttt attatactgg aacagctcgt 10500
gtcctctgtc tcttgcctcg gcgcctgggt ggcttgccgc ccacaggtat ttggtaactt 10560
ccctttctct tacctagttt tggtatcaaa gctaagttca cgaatgaagt gttctctcaa 10620
ttgattctgt tagaatttgt ctacgtctgg aattacctgt tctctatatc gtggaacttc 10680

cctgtaaaac cgtctagggg aacattctta actactgact caatttcatc agtgattaca 107
40
actcaggctt tctattactt ttagtcagtt ttggagattt ttcttccatt tactttgagt 108
00
tgttccaact taagctgcat gcttattttt ctttagcttt aaaaatacag acatacagcg 108
60
tatgtgtgta taaatggcac tatttatctg tagatgcaca tacatttagg ggctatgttt 109
20
ttttgtgctg ctttagctct gttccacaaa tcttaaattt tttcttttg agacagtctt 109
80
gctctgtcac ccaggctgga gtgcagcggc tcgatcttgg ctcactgcag cctctacctc 110
40
ctgggttcaa gtgattctcc cgcctcagcc tcccaagcag ctgggactac aggtttgcac 111
00
caccacaccc agctaatttt ttatttttaa tagagatggg gttttatcat gttgtccagg 111
60
ctagccttga actcctgatc tcaagtgatc cacccgcctc ccaaagagct gggattgaca 112
20
gcatgagcca ccatgccagg ccctgttcca caaattttaa tacgtagcat tttcattatc 112
80
ctttagtccc aaatatttct aatagccatt cggaattctt taactcatag gtaatttata 113
40
agtgtgtttt aaaaattcca aatatgaaat gccactgcac tccagcctga gtgacagagc 114
00
aagactctat ctaaaaaaca aaaaaaaata gctcaaattt tttttaatga cattgggata 114
60
cggtcagata acgacactga ttccttgaaa tatcttaaga cttcttttct ggacaaagta 115
20
ggtggtcagt ttccatccat gttccatata tgcatggaaa gagtatgtat tcttttcaga 115
80
tactgttgtg tctgtctctc agctcaagct catgctcacc tcacatccta tctttcacag 116
40
ggctttttca tcagttgcct ctgtctggta acagtcacac cagctcctgt tcagttacag 117
00
tctttgtctc ataaccggag agtttcatca aaagaatttt atttacagct ttatcatcca 117
60

tatgccacta aaattcacct gttttctttc aacctgcact cattttgatt gcctggaact 118
20
ctggatttaa ttcttccatc ccactttgta tcttgcattc acttcactct ctctccagct 118
80
tttattcttt ctttctcttt ccctaggtcc aatgcacttg acccaactca catgcgtgga 119
40
ctccgggaag gtactgctcc ctccctccaa attctgagca gtaaaatgcc gccccggggc 120
00
actggggaac agaaaggaat gagaccccaa caggcagaag ccaagagagc ggggaggagc 120
60
catggcgttc tgccccagga tgcaccacgc ctggacgtgc tccccgact cccagtgcca 121
20
ggtgcccata tgccacacct cagggttgtc ctctgttcgg gtgagctgcg gactaacgtg 121
80
gcccggcagc agaggccacc gtcttctgtc ccgtggctcc tgcgaacaca ggcagtgggg 122
40
aacaggcagt gactgcccac ccccaccgtt cctcctccct gaccctgcaa acctcgggga 123
00
agctttcagg cccgggaaag cagaccaggc cccagtctcc ctcacccttt ccctgggtct 123
60
gaaggtcccg gatcctgcgt tcaaggatga cgctgaaact ctctctttct cacatgggat 124
20
ctgtgatctg ggccctcaca actcagcaga gcaccactgt gtccccctca catggagagg 124
80
ccgaggtctg tggagatcct gggacagagc cagcgtcaag gactcagagg gtgtcctgga 125
40
gtctcctagg acggaagacg gcggccccag gtgggaaaga ctcagaccag gcctgcgcgc 126
00
tccaggtcct gcggcaggat gcggcccttc ttgcgggctc tgagcaggcg gcgctcggcg 126
60
cgggccgcct tcttcctgcg caggttctgc cgccgccggt cctggcgctg ctgcatcttc 127
20
tccaccacgc cggccgtgcg cttctcccac cggcgctgcc gctgcgccct gcgcttctcc 127
80
ttgcgcttca gggcctcctg cagcaggcgt tcgtcgtcac ggatcttcac gccctccgcc 128
40

ttgtagagga ggttggtcca cttcatcttc gcctccagct cctgcgcctt cccctcatcc 129
00
tggccgcgca gctcgtccag ccggctctgc cgtgcctgca ggcgctccag cagctgccgg 129
60
tagttcctcc cggtcagcgg cgtgaggttc cccttcaccc tctgcctctt ctcttttctg 130
20
cgctgcgcct tgctggccgg ctcgtcttcg ctcacctcca cctgggagga aggtatgaca 130
80
tcagctcatg ccagccctgc actaggcccc agccttggcc agtaccctaa gggacctgcg 131
40
aggtccccgt caccacctta cagacatgat ggggttcgga gagagatcat gaagctaaca 132
00
aggggcaacg ctgaggcctg aagccgaccc agcccgccca aggacccagc tcccgctcac 132
60
cttattgaag atcagcccgg gcggctcccg cggctccgtg caggccccct ctggggttgc 133
20
ctccaccacc tcctgggcct ccgtggcctc ctcagccttc ctggccttct ctttcgcccg 133
80
cagctccttt cgcttcctct tcttccggtc ccgttcctgc tttctccgcc gccttttctc 134
40
caaggcggca ggggacagct ccttggcact gccctggggg aaagaggcac ccactcatta 135
00
aagttctctc gatcccaggg tcccccagcc tggcccatag tcgagaagaa tcagggctgg 135
60
aaggcaggtg agaaaatcct cacgcaaaca aggggcccgc ggagttcaat gttccaccat 136
20
gatgttcccc aaaaagcaaa tgaccccaaa agagagaagg gacccccaaa taagaatcac 136
80
agcttccaat tcccgggtgc ttaccccgtg ccaggataca ttacgcacat ggtttcaaat 137
40
gtcatacatc gttttataga tgaggagggt caagaccact gcctagaact tggagttggc 138
00
gtctgaacac ctgtcctgac cgctgcccat tctgttcacg aggtacccaa cgaagccctc 138
60
cccaatggcc tttcccatcc ccgggccaac aagagcccta caccagccca aacgagacct 139
20

gtgcttcagg aacaagggcc cagagccttg ctcacctctt aggaaccaca cactgtacct 139
80
cgggatggcg gaggagagta gctggggact gtcccccaca cagcacgagg ccttaaggaa 140
40
gggccccagg aaaagggtgg gtaggggcca acacagggga gacagtacca ttcagcacaa 141
00
agagctgcat tggtgcttcc tgtgcctggc cactcagctc aagtccctac tcaactcaca 141
60
ggactattac gacattcaga gaaacagaca caggaggtgc ccatcccagt gtcagttcag 142
20
caaaggcagc ttcccagaag gaaggaggct gatgagcttg gggactgagg ttctccaaga 142
80
aaataactgc tctcccagag cacacctgct ggggccctgc cagactcgct gcagagggga 143
40
gaacaggggc tacggcccct cgctgacagc tgaccccagg gagaacacag gcagagcagt 144
00
gacggcactc cagcaaacct gcccgcctac ctggccccgg gcctcctgga tcttctcatg 144
60
cagtcgctgt cgcagaacat ccagagcaaa gacagactca ggctcagtgg ccaggccatc 145
20
tgcagggaag gagacaggac tgcaggggc cctctcttcc cctccccctc cctccctagg 145
80
gccacgaatc cctgtcccac tgtggccact catggatctg cagggcaatt ccagtaaatt 146
40
ccactccacc gcttcaacct ggactggttc ctacaacatc ctccagaaca ggtaactcag 147
00
tgcctcacga ggtaggtcac cgtggtatga gaaaacactt cctacggtgc aagccaaacc 147
60
acctcctcca aattttgtag cctgggcccc agaacaagtt ggctctcaca aggcccctgc 148
20
agagacctga gggcagggaa gctcttcaag ccaagctgct ccaggtcctc agagagacac 148
80
agccttgccc cctgacatcc tgctagccat gtggcctgga atgccacaca gttccttcgg 149
40
tcccatccac cctagcactc ctgtgatctt tgctcttggg gaagtctcgt gctatccctg 150
00

tgcgcctgct gctgcttttt ttttcttaag agcaaagggg accaagccca agcccagccc 150
60
cagccccgcc ctgcacagaa ccaacatgcc ctgaagcctc tcacctgcag ggttccctgc 151
20
tgagctggaa gcccaagctg cttcctcttt ggctgcctca ggcctcctgg ccccagaggc 151
80
tgctggagat ttctccccca aggacttggc cttgtgctca gcagccttct cttctcgctt 152
40
ccggaatttc ttttgtgttt tcttcctttt ctttttgggg ggccctgcag tttctgagcc 153
00
ttgagttttg ccagctgaaa tgcaaaataa gaaagagtta agtcccaatc tcatggccca 153
60
ttcaataggc aggaaaggct caccaaggct ttgatcccag gaagatgccg aaagaggatc 154
20
aggatcgggg gccagagaca ctgatcctag agctcagaac cacaaccttg acccagtagt 154
80
tctgcttgtg agaattcatc aggcacaaag atgaggcttc aaggacgttc atcaccatta 155
40
tttgagtgaa acattagaaa aacctgaata tccacctcca ctaacaattt ctggccctgc 156
00
tttacaataa gctaccatgc tgccatcaaa cacaatggca agggctttca ttttagctgc 156
60
tggggggttat gtttacattt tttcttttttt gagatggagt ctcaatctgt tccccaagct 157
20
gaagtgcagt ggcgcaatct ctgctcactg caaactccac ttctcgagtt caagcgattc 157
80
tcttgcctca gcctccggag tagctgggac tacaggcgcc caccactgtg cccggctaat 158
40
ttttgtattt ttggtagaga cggggtttca ccatgttgtc caggatggtc ttgatcttct 159
00
gatcttgtga tccgccctcc tcagcctccc aaaattctgg gattacaggc atgaggccgc 159
60
acggccggac aatgtttaca ttttaaatgg gagaaatcag tctaagtaaa gtaggagctg 160
20
aatcgtttta atgaaaaaaa aaaaaaattt caaaaaggat gagaaagaca gacggaagac 160
80

atactaggta atgagaaaat tcacttaaga ttttggattt gctataacca atatatcgtc 161
40
cttgaataaa aaggaaaccc cgttttacct ctggtggttc cagagaccct gctgctctcc 162
00
tctcctcctg ggcccagaca gcactgacac gtccccgctg tcggcttccc cacccgatta 162
60
tctacttccc ttgtgcagcg tggtcaccag gccatgagaa ccctaagcgc aggagccctc 163
20
tgccatcctc ctcctcccca caccgcacca tgcccgtagt gaaggactg aaagggtctt 163
80
ttcccactga ctgaccatta ctcctgtctc tactaaaat gcaaaaaaa aaaaaaaat 164
40
tagctgggcg tggtggcggg cgcctgtagt gccggctact cgggaggctg aggcaggaga 165
00
atggcgtgaa cccgggagac ggagcttgca gtgagccgag atcgcgtcgc tgcacgccag 165
60
cctggacgac agagcgagac tccgtctcca aaaacaaaaa caaaaacaaa aaaaacaaat 166
20
aagggaaacg gcgataattt cacaagtcac ttagattttt tttctagtac tttacagact 166
80
cgtctacacc ggctccggcc gcgtcccccg tttcgcaggc cccttagtcc cggcccggcc 167
40
ctgtgcgtta ccccgcgtgc gcgcctgctg ttccggggcc gaatgggagc agatcttctt 168
00
ggccaggctc tgcaggtagg cgtccttggc gagtagagag gccatggcgg agacccgggc 168
60
cgttcacgac tcacaccttc cccgctgcgc gtgcgactct caccacctcc gccggaaacc 169
20
accacacggg caggcgcggc caaacgaacg ccgagccgcc agcccgcgcg ctcgattagc 169
80
caagcctgac tccgccggaa gcggcgcgcg gggcggggcg cacagcattg cgggccgagg 170
40
acagccaatc tccgcccgga gtcggtgcag cagggcaccc ccggggcctg gcctcagtgc 171
00
ctctatcacc ccggtcccgc acgtgttcct gtgctcccct cacccccaac cccgacacag 171
60

caggcgctca tgagtagggg cgaaatgaat gaatgaccag cagtacattc attccgtcct 172
20
ttggagtggg gggcctcagg tctcaggcgg acacagactg agcgcctggc acgtggcagg 172
80
ccctaggttc agtcctaggg gacacaagca gtgtgtcaca cacacagatg tgttctcggc 173
40
gagtgtctgc cgtagaggtg actgataaac ctggcaagcg tgtgactgtc aggtgagggg 174
00
agcgccaaag aaagcccagg ggaaaaggga gaagtgttgg gcgggaggga gcctgcatgt 174
60
tcaaggaaga ccccctgggc agtggctttt gctctgaaaa atagaattac acactacgat 175
20
tccctccccc accccggtga cggagtctcc ctctgtcgcc caggctggag tgcagtggca 175
80
agatctcggc tcactgcaac ctccacctcc cgggttcaag caattctcct gcctcagcct 176
40
cccgagtagc tgggattaca gacgtgtacc accacgccca gctaattttt gtatttttag 177
00
tagagacggg gtttcaccat gttggccagg ctggtctcga actcctgacc tcaggtgatc 177
60
cgcccacctc cgcctcccat agtgctggga ttgcaggcat gagccaccgt gcccagccca 178
20
cactgcgatt tttaacacaa gatgtggtct ttataataac tcactagggg caggagaaaa 178
80
gagaggtcat ggcctgaggc ttgggaccac cacaccccag ttcctgcact ggaaaattac 179
40
aggctcatta aggacccaga gccccttcca gggccacccc gttggtagag aagggagaac 180
00
ttaagagtct taaattacaa tttgaaaagg tggctgctga tatatatatt agaaacagaa 180
60
ctgttgtatt tgaaacagaa ctaaactttc ttcccctaac ttctgccatg aggtgattta 181
20
aaaaaatttt tttggccagg cgcagtggtt cacgcctgta atcccagcac tttgggaggc 181
80
caaggcggga ggatcacttg agccctagag ttggagacca gcctgggcaa catagtgaaa 182
40

ccctgtctcc acacacaaaa aaatttaaaa ttagccaagc gtggtggccg gcccctgtag 183
00
tcccaactac tcaggaggct gagaagagac gattgcttga accggggagg cagaggttac 183
60
agtgagccaa gatcacgcca ctgcattcta gcctggagtg ccaggctgtc tcaagatata 184
20
tgtatttttt tcacttttaa aaaaggcagt caaatttagc agtgtggggg tcgaatgcca 184
80
actatagtga cactaaggtt aattagttct gacatcccac tgccattcag accagcctag 185
40
aggtgatgtt tcatggagga taggcgtgac gctgctgctg actcccctga actgctgggt 186
00
atcttaacac catcacctac tgagttcaga atgggcatct gaccccccaa gccccatctc 186
60
ttcctctcac tgtcaaccct tttttcagtt aaagcggctc catccttcca ggtgcttggg 187
20
ccagaccttg aaaccactga ctcctttctt attccccaca tccaatgctt cagcaaatcc 187
80
tgtcagctgt gccttcagag cacctccaga atctcagtgg acccactccc atctcatcac 188
40
cactttggtc ctgttttatt tattttttga aatggagagt ctcactcttc ctcccaggct 189
00
ggagtgcagt ggtgtcatct tggctgactg cagtctccac ctcctgggtt ctgcctcagt 189
60
ctcctgagta gatgggacta taggcgtgtg ccaccatgcc tggctaattt ctttttcttt 190
20
tttctttttt tttttgaggt ggagtttcgc tcttgttgcc caagctggag tgcagtggcc 190
80
cgatctcggc tcactgcaac ctccgcctcc cgggttcaag cgattctcct gcctccgcct 191
40
accaagtagc tgggattata ggcatggacc agcacgcccg gctaattttt gtatttttag 192
00
tagagacagg gtttctccat tttggtcagg ctggtctgga actcccgacc tcaggtgatc 192
60
tgcccgcctc ggcctcccaa agtgctggga ttacaggcgt gagccaccgc gcccggccaa 193
20

tttctatact ttttagtaga gacagggttt ctccatgttg gtcaggctgg tctggaaatc 193
80
ccgacctcag gtgatccgcc cgccttggcc tcccaaagtg ctggggttgc aggcgtaagc 194
40
caccgcgccc ggccgatttc tatacttttt agtagagacg ggtttctcc atgttgccca 195
00
ggctggtctc aaaacttctg accacaagtg atccacccgc tttggcttct caaagtgctg 195
60
ggattacagc aggagccact gggcctgacc tggtcctgtt ttagatttta agactccaaa 196
20
ataacaacca aatgcaacac acaataaaaa caggcataaa agcctttcag ctggaaccgc 196
80
caccttccag taattcgcca aaatgacgaa cacaaaggga aagaggagag gcacccaata 197
40
tatgttctct aggcctttca gaaaacatgc agttgttcct ttggccaagt atatgcaaat 198
00
tgatgagaaa ggtgatattg tagatatcaa gggaatgggt actgttcaaa aaggaatgcc 198
60
ccacaaatgt caccatggct agactgggag agtctacagt gttccccagc atgctgttgg 199
20
cactgttgta aacaagtaag ggcaagattc ttgccaagag aatgaatgtg catattcagc 199
80
acactaagca ctctaagagc cgagagagct tcctgaaacg cgtgaaggaa aatgatcaga 200
40
aaaagaggga agccaaagag aaaggtacct ggattcaact gaagcgccag cctgctccac 201
00
ccagagcagc acactgtgag aaccaatggg aaggagcctg agctgctgga acctcttccc 201
60
tatgaattca tggcatcgtg ggtgttaaaa aaataaaaga cctctggact agaaagaaaa 202
20
aaaataggca ttaaaaaaac tatttgggga acaactgaag aaatctgaat acagctagat 202
80
accagaggat atgcaatcat catccattct ggttgtggtg atggacgaag gagaatgtgc 203
40
tcagagaggc aaactgacaa gtacttccat gagttccact gccctcaaaa taaaaagctt 204
00

gggataaaag agttacatgg actgagaatg ggccggggct tagcaatgtg gaggccactg 20460
gtgaccttaa taggtgtagt tttgctagag tcatggagac aaaacctgtc tgacgtaggc 20520
ccaagagaga actgcaaatg gttatcagct gcttccccgt ggagctctgc tgccaaggcc 20580
atgagctgga gaggaaaaaa ggtcagcaga gcggcttggc tttaagaagg agaaataact 20640
tgattttcac atgggaatga tggtcctggc caagcagaaa atgaagctga tggcaggtct 20700
tatcgctcag gattttagga acagggtaag tgggatccca ccacaaagtc tggccagttc 20760
cttccgggct ctcgcttcat atatgcttcc atctggttgt aatctttttc ttcctcagtg 20820
gttaagtaca aggcatttcc acagctaaaa tagtgcggga acagatcacc acacgtatca 20880
atggggaaat tctccctcag aggggaagca acttccctag gccaccccag aagccaggtc 20940
cagagccagg actgggcctc aagctcctgt ctgtctggtg ccttctgctt ggctgtgggg 21000
ttctctggtt caggtggtga tgggtcagct gtgttcttac ctgtccccaa ggctggatcc 21060
tgggctgtca cctcattcat acatcgggaa agatgatggc ctccccccatg agaagatgaa 21120
gcacatggat ggtgctggag gaactggggt ggggtgctcc cccacacttc ctgtggacga 21180
actgtgctcc ccccacactt cctgcccagt gttgtgactt ctgcatttct aaggtgagcc 21240
tggcaccaaa agacactggg tctagacttc catcaggttc aagttctgat tcctgccact 21300
tcacttccta gctctgtggc cgtggtacac ctcacccctt ctaagtccac atcctctcct 21360
ctctaaaata ccggcactag gagcaccggc tctggtagca gtgaggatga gatgccagca 21420
gaggcacagg tgcctggatg tgcaatgatg gcagcaggtc cattcactga atactgggga 21480

cctaccaggt gccaatgagg agagagtgac cttgggccag gctgtccagg cagagctgag 215
40
gagctgctgc tcggacaact gctgggagac caaggatggt cctggactga caaggaatga 216
00
ggaagaaaga gcagcaatgc aaaaaagtgg cggtgtttta atcaagtctc attacaacgg 216
60
cagaattagg aatgagtccc acctagcttc cacatgcgtg ggcagcagct gtcacacggg 217
20
cctgtcgtgg cactcagccc atggcacaga catatgtggc tcagggcaag aaccagtgga 217
80
tggggctctg cacaggaacg ctggcctcgg gatgggagac ccggcctgcc caacactgct 218
40
cagagcccct cccaactctg acaacaggct cgggtcagga ctccccgaaa tcaggcaccc 219
00
tcctgctcca ctgctgagat ccccaacggg ccagacctag cttggaaact ttcttccacc 219
60
tggctggcca ggaaggcagc aaacagagat gatgactcgg aatgatgggc tattcggaaa 220
20
tggctaggga aacaactgtt tccctactgt cctggcggga cccaccctgg gctaacgggc 220
80
ttcccaagga agtcctagtg gctctggggt cctgaagtcc ccaaatggga acctaggctg 221
40
agagaagcaa ggctgtgagg gcatccaaag ggctgcctca ggttttgttc ctgagaacga 222
00
agcgtggccc cggaggctca ggcgtgctca gtggggccag ggccaccagc atgggagtgg 222
60
gcaggggctg ccacctgtag ggggccagca ctgggctccg gggacgccag cagaggggcc 223
20
gagaggccat cagcagagtc tgtgtcgagg tccagcctcc cgtaagcttc gcacagaggc 223
80
aggtcattag cttcgcaaag gcttgagctt ttccaccacc agaaacccca gggagagaca 224
40
ggagcaggca gagaggaaag ccaggggagg ggagagcaag acagaagcag agttaagaaa 225
00
acacgaccac accccagacc tgcctttccc tttctccact cctgctccat ctgtcccctg 225
60

agttggcagg cctggcaagg agaggcggcc agtggtgaga gccaccctag cattctgaaa 226
20
ggaggaagcc gccctggccc aaccaccaac agctgtgtga cctcagcagg cccttcctgc 226
80
ccagcctcca cgtgggcctg tcctagccta tgcctcccag ctggtgctgg tccccctcct 227
40
cttgcaaaaa gctgtcagcc tggccaccct ccctcctccc tgactgcaga aaccccagtg 228
00
tcactacact gcaaagagct ctctgagggc agactgtgtt ctttctgggt ctgtccctgc 228
60
ctgagactta gcctgaaggg ggcgacagtt gggaacacag gccctggcac ttcccgatag 229
20
ccctacatca gccaggcggc ctccagcagg cgtccccacc tctaacacgg ggaatcctca 229
80
cggcagccag gatgcaggtg aagagctcag ctagcccttc ccatgcccgc agctgtgaca 230
40
atccacgctc gcctctttct aacctgcagg caggggccct ggctttcccg cggcctgcct 231
00
cgcccctggc ttcctccctt gctcctgccc tcagtgttca gtctcagaac cgtcctgggc 231
60
acagagtggc atcccgagga aagcaaagga gaggcagagc cagaaagagc aactaacaag 232
20
cagagagggg gcaggcgagc acagaggcgc agctcatgcg gaacagggca gggcagggca 232
80
gggagcggag cgccctttgg gggaccagca agaaggggca gaggaaactc ggccaggacc 233
40
tggtcgctta aaggcaatgt acagaggagg gcaactgctt ctgccacagg ggctgtgtga 234
00
ggcccccag ggggcgctgg tgtggacagg aggcctgcgg gaggggacac aggctggcct 234
60
ggaagccccg ctgggtgaag ctgagggact gttgggggag ggcatgggaa gcctggcaca 235
20
gatgtcctgg gtttgcgccc tgctctgctg cagggccgtg agcaggttcc ctctctccct 235
80
gccccaaggc aaggcaggca ggctgaatta caggcccaca gctgctctgt gcaggggccc 236
40

tcagggacgg tggctgcctc aaagagaccg acaaactgaa cagctgtgga aggagatgcc 23700
cagaagggtc tgaaacaccc taggacccct ctcagccacc ctagtgtgga ggaaatgctg 23760
cctcatgtct gctgagttaa tgagtactgg ggaccaaaga ttccctcaga accccctgga 23820
aaactctagg actgctgcag ctcagcagtg ccactgagcc cagggcaggg aaaccaagcc 23880
ccaggtcatg ctggctctgg atatctgtgg ctcccagcat ggccaatggg cagaaaggag 23940
tattttaaat ccaccaccag gaaggaaagg tttgtccagg agaaggcaca agatggacat 24000
ggactctgca tctgctggac tgaatcttga ttttctaagc taatggggca gagtggaagg 24060
cctccctgtc tccagcctaa gtttccccct aagtaaatga tacgtagact ctgatttctc 24120
agggcccctc cagctcaaac ggtcgaaggt ttcagcttct tacggagccc cacaagggtc 24180
aggatgatgg gttttgaccc ttctgcatcc ctgggaccca gcacggggcc tggcacgtag 24240
gttggctcca gctccgtgga taaggctgag tgggtcacga attggagctc caatggcttt 24300
caaggccatt ccctgcctgg gcttcatccc ccacattccc atgactgtcc ctgcctccac 24360
actggggttt ggtgacatcc acatggaatt gcatccctgg tgccctggac ataggtgtgg 24420
acctgactgc ccaaaaaagg ctggcccagc cttggctgaa atctctacat atggattcag 24480
aaggtaacag gagcagcaca gccccagaac gcactgcccg aggaggaagg ctctcggaag 24540
agggcctgcg ggggatacag ccaggcgcct cccttctcca gactcggcct atccgactgg 24600
cgtgagtcct gtggttttca tcacattgca ctgtgggaaa ggcccagggc cctggcatat 24660
ggatgtgaat tgtctgtcgg tcaaacgctc tggccaggag ctctgctctg aaacccaggc 24720

atggctccac tgctaagacc agcaagaatt tgaaaaagca gaaggcagcc ctgcctgctg 247
80
gccaggccca ggtaggcagg ctcccgctct gcatggggag tccagcgcta tttatacctg 248
40
gacgagtaat actcctcctc cagctcgtag aggtcaatgg agatctcgtc tcgcagcgtg 249
00
atgagcttcc ggtcgcactc ctcctgcagt gtgcgcagct gctggttgcg ctggtcaatg 249
60
gcctcgttca cggaggggaa gtcattgagg atcaggaact gcttgaggtc ggacaccagc 250
20
ttcatcaggg actcgccggc tcggacctgt ggccatcaga accagggcgg gcacagggtg 250
80
agggggggaca gcggccttgc ctacaagtag ctgatgctgg gcaagggatg gcctctctag 251
40
gagcctcagc ttcctcatct gcaaagtggg actctacccc tgacctccac aggttcatgt 252
00
gtgagaatta agtgagaaaa ctgggatgtc agttctgcag ggcaggccct cccctctcgt 252
60
cagcctctgg cttccactcc tgcaggactg gcctccagtc caattatgca ggtgtacatt 253
20
ctgctcatga aaaactcaaa ccccacagat aaaactgcga tcccattttg cctcctgccc 253
80
cggggtagga ttaacatggc tgccagctgg gcagagctcc tgtgggacct ttttccatgc 254
40
attttgagcc ctggacgtgc acctgcggaa ttatctgctt gctgtgtttc tcctgtgatc 255
00
cgaaggaatc caatggcgcc tacagttcca ttcggctgtt tgtcttttca ctcacaactg 255
60
accttaggaa ctcctgagtt agcaggaagg gctaccctct ttctggccac tgcacggcta 256
20
caggacgggc atgggtggct ctctgcccag ccctcctcca agtgccccac gtgaggacag 256
80
tctcctgaga aatgatttgc ccccacctca ttctttaaag ctgccgagtg gtgaaaccaa 257
40
agcatctcta catttgcctc cttctgctcc ttctcggggg aagaagacca gaccagcccc 258
00

agagcccccct ccactccacc caggaaacct gaggggactg atgctcttgg agcccaggcc 258
60
gtgccctcca ccctggccac tcacgatgtt ggcggctcgc acatgcatct cgtaattgtc 259
20
ctgttcaccc tgagtggccc gtgacacctg cgtctcgtcc tcaatctggg ggaaaacaag 259
80
aaaacctaga aatcaaccca gccaaggcat ccccacccct ggcccggccc agcccagctt 260
40
acggtaactg tcatctaccc aggatgtcca cactggccac aacctgtctg tggtgccatc 261
00
tacagaccta agcgctgtca gccagacctc tgacaaggtt caattccacc ctctcttgtt 261
60
ctggccatgg gagacaacat gcacctttgc tcaagacaaa tagaggcaac ccacgctgct 262
20
tgctgtacta ggtgcaggga tggggcttta tcttggcccg gtctttttgg gctgtgggag 262
80
ccctgtttgg taggaaccgt tgtcacttgc ttggcatttg gtaaactgtt ggatgagtga 263
40
agggatgatt taagtttttt ttcgcgacca tttcctgatc acttcccagg tgccaggctg 264
00
tgtcatgcac ttgaccacca ttaacccatg ccagtttcac agaagcctgg cctgccaccc 264
60
ctggtgtata ggtggccaag cgaggctgga tggggcttcc cggcaggcct tgcaaaggct 265
20
cagttcgggg tgggtgtcag gtaccctcag tggttaagac ccagacttcg tgggttcaaa 265
80
tcctggctca gccagttgtg agctgagtga ccttgggcaa gtcactaagt ctcctgggcc 266
40
tgggtttcct cctgaaatga agaggacaac agtgcccacc tcacagagtc ctcatgaaga 267
00
ttcagcagaa caggcacaga taacctcaca acgtggcttg atacatggca agcactaagt 267
60
acatgctagc ttccatcctc atcatcatcg cttatggagc ctgccaggtc tgcagcagcc 268
20
aggagggatc cagcccaatc tgatgcagct gtagctgcag tggtcagcag gcttgcaggc 268
80

cccagtgctg accgctccac ctgcctggag gccccccccc acttctcccc tcagtctgct 269
40
tttctcctgc tcattggctg tgtgccaggc cccgaggtaa ctgttttaca cagataaact 270
00
caccagtcat cacaaggacc ccaccatgca ggagaaactg aggcactaaa aagtgaaatc 270
60
tccatccaaa tccatactgt tagtaagggc tggacgtgcc attatttggt ttgcttatct 271
20
actgaccagc tccctgtgca ctgggtgctt cctgcctaga acactattca ctcgcgctgc 271
80
tcagcccctg gcctctcctg tctccacccc ttctcagact ctgctcccct tggtgggcca 272
40
ccccaccccc accttggcgg tcttgatgat ctcggtgaag ttgtccatga tggacttaat 273
00
gtcgtccttc agccgcttgt tgtaggactg cagcagcgtc tccttgctct ggggcagggc 273
60
tctctgctgg gccatggccg agcctcaagc agcgcagcgg ggagacctgg gacctagagt 274
20
gcagcacaga cctctgagtg caggcagagt ctaccccagc caccctctat gccccaacct 274
80
tagcagaaca cgcagatttc tggggattcc ctttctgcca aataaagtca atcactgaaa 275
40
cacagatgaa ctcattccat cagcagacac cgcagggggt gccaggactg gccccgcctt 276
00
tgccagagca agctcatcct ggaactcctg gccagccctc caagccctgt ccagggctcc 276
60
agtccagact tcacgtccag gcccacagcg tgtcccaggg agccctgcaa actcaacacc 277
20
agctccccgt ccccagccct tggcttaact tcttagcccg ccgccacctt cgccacgccc 277
80
tcccatccga agagtccttc caattcgacc cctctgcacc gcctacatcc acgctttcct 278
40
tccactccca ctcaggctgc cccgctccag acctcatccc tgcactgcgg gccccgctcc 279
00
ctccagtctc tgcgcggcag ggaagaggtc ctaaaaagtg gtcattccaa ccgggcgcgg 279
60

tggctcacgc ctgtaatccc agcactttgg gaggccgagg cagggatcac ctgaggtcag 280
20
gagtttgaga ctagcctgac caacatggtg aaaccccaac tctactaaaa atacaaaaat 280
80
tagccgggcg tgatggcagg cgcctgtaat cccagctact cgggaggctg aggcaggaga 281
40
atcgcttgaa cccgggaagc acaggtcgca gtgagccgag atcgcgccac tgcactccag 282
00
cctgggcgac agggggagac tacgtctcca aaagaaaaaa aaaagggggg taattccact 282
60
ccctcgctta acatccctca tggttccccg gtgcgccggg acaaggggct caagttccgc 283
20
gccgcgcctc tcggcctctg ccctccagcc gcactggacg gcctcggcgc tggagttgcc 283
80
tggccctggg gccgggcctt tgcgcgcggt gctcagggag ggcccggggc cccctaggtt 284
40
cggagtctgg cgcacgaccg agcggactcc tggacgcact cgcattgttt gtgcccattt 285
00
ttggcggggt gtgggaaata agtcacacgc aggaaagggg atctccgacc ccagcgccta 285
60
cgcacccacc cacccccact cccgcccaca cacccacccc ccctccatcc ccacccccca 286
20
ccacaccctc atacccgccc cagcgcccgc acaccagacg ccgcgtccgc cgggtcggcc 286
80
tagggcgggg tggtcaagtg cctctgcgac ccgcactttc ccgcgtctct cccacggcct 287
40
ggccctcccg ccgcagtctc tcttccccgc cgcgccgcgg tccgaaaacc tagtcagccg 288
00
ccgcagcctc tcggccccgc ctcgattttt agctttatag gaatgctgtt gctttaaatc 288
60
cgaaatcccg tgccggtatc aactctcgcg atctccgagg ccgcatacat attacccaca 289
20
attccctttc ctttctctct cctcccgccg cccaagatgg tgagtgagct gtagttccgt 289
80
ggcactatag ccaggttccg gctgtatccg ctgccatcct cctccaggcg cggcctcgga 290
40

gggcctcctg ctcctcctgg cgctaggaga gccccactcg gtgtggcacg gagacaccga 29100
ggtggattag agccccactt ggtgtggcac ggagacattg agatggacta gagccccggg 29160
cggccgagag cggaatgcgt tgttcccggt gtcgcagggc tgggtgtcgc aggcctggag 29220
caccgcagtg cggggctcgg agccctagcg tctctcgggc ttgctggggg ccgctccaga 29280
ggccttgtga gcgacgagtt ctgagcccgc ccctgttgct tctagagcct gtggggccgc 29340
gactcagagg agtcatgagt ccggggtgtc tcctgggtgg gcgacgcgaa gagagcgtgg 29400
tctcgggctt agccttgctc tggccactcg gggttcccgg ggctgcatgc ttgtgcggct 29460
gaatgtgaga tgctcctgtc gaggggtggt gctgggggt tgcagaaagc tgctcgccag 29520
cttagttcag gcaggtgctg tcagcgtccc ttgttttgga ggagccagcc tgagccctac 29580
ccccgacgaa gcgagtggag gcggcggttt aactgacgtt ttctttctgc ccagccgaaa 29640
ggaaagaagg ccaagggaaa gaaggtggct ccggccccag ctgtcgtgaa gaagcaggag 29700
gctaagaaag tggtgaatcc cctgtttgag aaaaggccta agaattttgg cattggtaag 29760
taacaaacgg cagaatgaaa acggtctatg tttttctcaa gggaaggtgg taattgggtt 29820
gtgttgtatc ttgtaggttt tagtgggtgt aaagtggtcg cagtccttaa tttgtgtctc 29880
ttagagacgg gggcaatgat acatgcttct tgctttcatt gggagttgct gagcgagcat 29940
tcagctcaat atggtagtgg ccttgaattc agcttagcca tctggaaaca agtacagtag 30000
cagtgtcgca gcgaggtact aggactgcaa ttctgctgta cttcgtggca ccttggcttc 30060
ttgttagatg aggaaaagca tcgtgctctt tgttctcagg tgtttgtgtg cagatgatgt 30120

aaaagaatat ttgctatctg agagatggtg atgacatttt aaaccaccaa gatcgctgat 301
80
gcaccaacac ccttcctagt ggccccagac atgaacttga catggaattt gagcctcact 302
40
cggtgtcacc ctttacttct caggacagga catccagccc aaaagagacc tcacccgctt 303
00
tgtgaaatgg ccccgctata tcaggttgca gcggcagaga gccatcctct ataagcggct 303
60
gaaagtgcct cctgcgatta accagttcac ccaggccctg gaccgccaaa caggtgaggt 304
20
tctgtggcgt ggaaaggagt ttctcaggca aggattcctt atttcatcca gaacatgagg 304
80
gggatggtct taggcttctt gaactgcagt tgtcattaaa ttatagtcat atagcaggac 305
40
cgcagtccag catttgttat taagtgttaa gtgacaagga ttagaacctt gactccaagc 306
00
ctaaactgaa gagtgttttt ccagctactc agctgcttaa gctggcccac aagtacagac 306
60
cagagacaaa gcaagagaag aagcagagac tgttggcccg ggccgagaag aaggctgctg 307
20
gcaaagggga cgtcccaacg aagagaccac ctgtccttcg agcaggtgag taggccccac 307
80
cttagggtga acactggggg cgggctgttg cagtgatgta aaatttcttg gcctgaaatt 308
40
actgtgaaga gtaaaaccga gctttttaac actgagtcag cagctgagcc cagcagcttc 309
00
ttgtgactag agcaggccct gtgagtgctc acaaagtggt tgtgtgttct aggagttaac 309
60
accgtcacca ccttggtgga gaacaagaaa gctcagctgg tggtgattgc acacgacgtg 310
20
gatcccatcg aggtgcgttt gcctgttgac tgctaaccca agggcttctg gcagtaccag 310
80
gaagagagag tagacctaat gccaagtcag tgatgggacc gaagtgggtg agggcagtac 311
40
tgacacagat ccaacacatg cgtggctctt gcaatgatgt gaatctctca ctgaattcaa 312
00

ccttgaagtg cgaatccatg agctttttaa ccctgagcaa ttgttacaag ctaactgaaa 312
60
tttgctgctt ttggtcaaaa tacagtcttc agctaatgct ttcttccagc tggttgtctt 313
20
cttgcctgcc ctgtgtcgta aaatgggggt cccttactgc attatcaagg gaaaggcaag 313
80
actgggacgt ctagtccaca ggaagacctg caccactgtc gccttcacac aggtgaactc 314
40
gtaagtacac agcctggccc caaacttccc cccagttcat ttaatccatg cctcacagtt 315
00
gtttcctttt gccttaaagg ccaatctttt agtttaagaa atatatttat ctgaactttt 315
60
gccaatgatg gttaagaatt tcttcacctg aataaaccat gtggtcagca ttgcatctga 316
20
ggcaaaagac tgtcttgagc taaaaggtat ttttgcattc taaaagggaa actaaggcaa 316
80
aaaacccact tttgtttccc ctcctgcctt ttagggaaga caaaggcgct ttggctaagc 317
40
tggtggaagc tatcaggacc aattacaatg acagatacga tgaggtaaga ggcagcttta 318
00
caccaaaata ctgtcattca caaatctttc tcccaaataa ctggctggct taacctatga 318
60
gaagttctat ctgacgatca gcttggaaca gccaaacaga attaacgcaa ctaataacct 319
20
tgaaaatctc agaaaacagt aagccaagct aactgcctct ttttgtcttt tcagatccgc 319
80
cgtcactggg gtggcaatgt cctgggtcct aagtctgtgg ctcgtatcgc caagctcgaa 320
40
aaggcaaagg ctaaagaact tgccactaaa ctgggttaaa tgtacactgt tgagttttct 321
00
gtacataaaa ataattgaaa taatacaaat tttccttcag ccagtgtctg ttgagtatct 321
60
cgggttgaat cttacttggg gttagcaagt atctttttga gacacagcct cactctgtcg 322
20
cccaggctgg agtgcagtgg tgtgatgtca aagcaacctt cgcctcccag gtttaggata 322
80

ttctggtgcc tcagcatccc aactggctgg cccatatttg tgtttttggt agagatgggg 32340
tttcaccatg ttagccaggc tggtctgagc tcctgaccgc acccggccct tcccaccctt 32400
aaatacattc ttaaaccagg cattttgttc cctagagatg taacttgagt atcaagtttt 32460
gggaaagttc tttggactga aaccaagcca ggattttatg atgaaccagt catgagctca 32520
tttaaggtag aaggccagaa ctttatacca gtagcacatg atccagcata aaggcagtct 32580
tgaaatactg cattatccag ggacagggct tcagcagctg atctgtcaca caccaggtgt 32640
cccacgtagg aatttcttaa accacaggta ggatgtagct gcagagagtc catacctagg 32700
ggttgaaagc aagccagcat tagcaggctg ctaggctgaa ggggaggaag ccagagggcc 32760
gctggggact caccaggtca cgatgtactg cagatgctcg ttcctcagag taactctggt 32820
ttgccctcca atgggtcctc cagggactgt gctctctgtg gagacagcag actcaagtcc 32880
accccctact ggcctgccag cctctgcacc acttcctagt ggctgtcatt tttgcgtggc 32940
cagttggaag tcctgtatgg cctttacgtt gggtgaccat ccccgccctt gtccgctcag 33000
tacttgccta ggttctttgc tgagttgctg cctcctccca cccgccatat acacatgtga 33060
gaacataagc cacagtagtg actgggcaat gagggttagg aggaaggaca gtattcacaa 33120
aagctacttg ttccgagatg ggctggtcca caacgtacgg aagttggcat caatgaagat 33180
gggctctgcg cctgtgattc tggccatagc ttccaggtct cgataatgcc agtggttcct 33240
ttctggattg ttctcaggga caaaaggctg cctggtttct gtcagcctca ccatcccaat 33300
gaggtccact tctccctcaa tctataaagg aaggtgtgtg agattgcatg gagcctggtg 33360

gactcccaga gccttctcta aagtaggaag agtccatgtc ccttacctgg cctttctgcc 33420
gggtttcagg attcactttc ttcctgggaa cgaaccctct atttaccagg atggtgactc 33480
tagggtaatg aaagtgctac ttcaggtggg gagggttttt gactaaagac agtcactcat 33540
ggtcactcag gcacccatag gaacaactag agcaccaagg aaggctttta aaacagggct 33600
ggctcagtgg agccctggca gtgccacaca ggcaaagtct tcctctcttg aggcaccttc 33660
gtggttggta aaaggctccc tgccaccatc actacctttt taccagtgtg gctttcccct 33720
tctatctctg cttcttgtgg tctacctact acaacgtgca actggtgagc aacgctgcca 33780
cgccagagtt tagaccccac acctctcccc tgaactatgg caagagctgt ctccaatccc 33840
tcctcctaac caaggcagcc gtgaggagca gccctggcac cccagcctgc tggagatgag 33900
tacttgggcc ccatcccagc cctaaaacag gaacccatgg gttaacaaga gccccaggta 33960
ttttcatttt tacgtgaatc ctccagttcc ctagttaatc acacaggtgc tcctgtacct 34020
gtcatttgct ttgcctggaa tgttcttccc atctcttaac tcccaaatag ccctctaggg 34080
agccacccct gcctccactc cctcaaggta gggctggggt cctttctctt gaagtcctct 34140
tgctggcccc tcatagcttg ccatcatcta atgtgtgggc aactagacag ttctccagag 34200
gcagggcccc tgtttcacga atccctcctt tcccttcaga ggtcaacata cagaaattat 34260
ccagtcataa atgagctggc tgagaagatt aagtcaatgt cacaattagg gacttaaact 34320
atgcaaggaa ttgaatctcc tgggtatctt gggttcccca gggtcctacc aaggttgggg 34380
attgtgaagg aaatagtgat gtaaattagt ataccctgac tgcctctgcc aggacagcca 34440

gctcccacat gtcctactca ccccaggtcg gtgcagtgga agggagtgac cacataggcc 34500
ccactctgag ttgaggagga gatgaggccg ccctcccggg cctcccggac agggtccacc 34560
atggtccggg gcatcatata cagctccttg gaatggtcaa agcaccccct gaccttcact 34620
ggcctatact ccagattttt cagttccatt gggctgcatg gagataagaa cagtggccga 34680
gcaaggtttg gctggaaaac gaatcccctg agggtggcag actacacagc ccaccagggc 34740
cagacaagtg aaggagaaag gcaaagggcg tgctcttcaa aggggaactt tgatgccatg 34800
tgggaatgtg gatctgcact gccagagtcc aactttacaa gagaggctaa aaatctggac 34860
tcctcaatga atattttatg tgaaatttta aaaaatatgt gggccaaaac cccatctgca 34920
agccaaatct ggcatactgt ttgctaccct gaatggaaaa tgtggctgaa tatacctatc 34980
tctgtagggc atattctagg gagagagcag acaaatcatt cagggcactt tttcaaacga 35040
ccaccctcac ggtgagacct acattatctg tcctcagcca cagggccttg ggccctgtgg 35100
aggataagtt tactatacct gaaaaaaggg tgacacccag gactcaaaat aagactttcc 35160
tccatatgtc aggaggcggt ctcaggtagc ttcacaaggg cagtcaggtg tcaacagcaa 35220
gcccaacaga tgactgataa tgagagctcc cacctgaggt caaggcagtg actaaaagtc 35280
ccaccaaaag gggcaagctg gccagcagaa gccagggctc tgctgttgaa ctcaagtaaa 35340
acaggcccta ggggggcagc catgcactca ctcggctggc agagggacag gctcagccag 35400
aactctggac tccaactctg caatcaggtt cagcttccac ttccgacgct ggacctacag 35460
tgacagagca taaggccaag cagatggcag caaggtcaag ggcccagagt tacgcacacc 35520

agatgccggt ctttacctgc catgtcccca agccaaaggc agtcacaggg atgaggagca 355
80
ggacccactg aagaaaggag tcatcttccg cttttgtggc agatgcttct gctgcagaac 356
40
tgccacatct gcttggcctc caggccaccc ctggagagtt tcacaacact gacatggagc 357
00
cagaagccct cgaacacaga cctggagcag cccgttccaa gacagactcc agtactgcca 357
60
atcaaaacct gctgcctaga gccaactagc agcacctgta tccagcccag ctcctaaccc 358
20
ggtgcccaga acaaggcaca cacagtacgt ctgccaagtg agtaagtggg atctagggct 358
80
cagtgccggt ttgaccacca accacgaccc tgagtaactc atgccttttt actcaagaat 359
40
ctagatgtat tccactgcac taagatgcac cattcatcca ttcaaaatgc atttaataag 360
00
cagctacagt tggccgggca cggtggctca cgcctgtaat cccagcactt tgggaggcca 360
60
aggcgggcgg atcacgagat caggagttcg agaccagcct gaccaacatg cagaaacccc 361
20
atctctacaa aaaaatgcaa aaattagctg agcacgatgg tgcttctgta atcccagcta 361
80
ctcgggaggc tgaggcagga gaatcgcttg aacctgggag gaggtggttg ctgtaagccg 362
40
agattgcgcc actgcactcc agcctgggca acaagagcga aactgtctca aaaaataaaa 363
00
aataagcagc tacagttact aagtagctta gtcaggcact agtggttgtt gtgttatgta 363
60
gaaaaatata tcaattcagt aatggctcac agagctctct taacagcttt ccatttttac 364
20
aactcatcgt aaatgtaaag agggaagaat gtacacagaa agtgccttta agctctcaga 364
80
ggatgagttc catccgctga aaagcaccag gggccttgcg gatactattt tttttttttt 365
40
ttttataaaa aggcaggcct ggggcaggct tgcacagcac tctacacacg gcaaaacagc 366
00

cctgtgctgc acactgagag gaacacgtaa gccgcggtgg ttagatacgc tttactttca 366
60
gagccctggg ctttcagcct gggctggcca cccattagct ggatgggacg ttcggaagta 367
20
actctcgagc cttctctgta acaggggaac gaccaaggag ctacctctcg cgttgtgagg 367
80
acaaagcgct cgctacatgc ccggcacacg accacaattc cactgaaagc attttaatac 368
40
ggaacttgtc actcccaggg agcctccgct cagccggcag ttggttcatt tcaatcccca 369
00
cgacaaccct tcaaagtgca gggcagacag caggtggctc tgcccaggcg cctggatcac 369
60
agcccggcct gcagccctca cctgggcgcg gggagaccct gaggacgctc ctccaggcgg 370
20
cgctggccgg ggcctgcgga cacggacggg cgggctgagc tccgggaccc ctccccgcgc 370
80
cccgcacccc gcaccccgca ccccgcaccc ggcgctcacc cgtcccagcc ccgccgcccg 371
40
cagccccagc tgcaacgcag ccaccgccgc catcgcaccc ggccccgcgg gcgcttccgg 372
00
gacgcaggaa gcatctgcat ccggggcgcc gctgagtccc gcccagagcc ccgcccccgg 372
60
ctccaggttc tgcgagcggc ttccgccggg ctgctccgcg ggcgcgtcgg ccatgagcga 373
20
gttgccgggc gacgtgcggg cgtttctgcg ggagcacccg agcctgcggc tccagacgga 373
80
cgcccgcaag gttcgcagcg cgggagggga acggagtggc ggagaagggc gcagttggga 374
40
tgaggggctg aggggagggc aggggagagg agaggacagg ggagagggga gaggggagag 375
00
caggagagag gggagggcag gggagagggc gcggcgggat caggggagga gagggaaggg 375
60
ggcgcggcag gagggggcac cagggagcgg agccctggcc ctcctgacgt cctgcccgcc 376
20
cacgcgtccg caggtgaggt gcatcctgac aggtcacgag ctgccctgcc gcctgccgga 376
80

gctccaggtc tacacccgcg gcaaaaagta ccagcggctg gtccgcgcct ccccggcctt 377
40
cgactatgca gagttcgagc cgcacatcgt gcccagcacc aagaacccgt aggtggtccg 378
00
cggcggcgcg gggaggccca gggcaattag gacagcccct ccgctggact ccgccagtgc 378
60
tgcagcccct actctttcag agttgggagc cctgggaccc aggtgggcgc ccgggtgctg 379
20
gaatcacctg cggtcccagc ggcgaggcct cttggtgagc tcgtttgctc acctgaggtt 379
80
tgtcctgtgg ggtgtggctg cttcccagat gagtagaggc ttgtgatttg tcacctgagg 380
40
ttgtgaacag cgttgggttc ttcccttaac cccagaaggg gtctttgatt tagctgggag 381
00
ctaggctttg taataatcgt caaaacagag ataggatgtt tccattcatt gagcccttgc 381
60
tccaggtgga gccatcctct tgcatgaact catcctggag cagtcagtga ggctgccatg 382
20
cgtgcttttc cggtaaacat taagaggctg cagtcggcct gggtcagacg gttccccacc 382
80
cagcttcaga gtggaattgc tccgggagcc tttaaaagcc cgatgtccaa gccgcatggt 383
40
agactgtcca gggatgagtc caagacacag ccaccagtct gaatccttgc tgtgaactgt 384
00
ccctacaaat ttggtctctc tgctctgtag gcaccagttg ttctgcaaac tcaccctgcg 384
60
gcacatcaac aagtgcccag aacacgtgct gaggcacacc cagggccggc ggtaccagcg 385
20
agctctgtgt aaatgtaagt cccagtggac ccccatcagt gcatcgccat ctgagtgcat 385
80
gcccgccttg ccccagatgg agcgtgcttg aaggcaggtc gtccttcagc gatccgtgtt 386
40
gatgcatcag gcctgttttt tggcacagtg aagagaggga tgatggcccc tgaccccaca 387
00
gcttttagtg caggcaggca cagagccaga agcaggctcg ggagtgagtg agtgtgcgtg 387
60

gcaatgcgag gtgtgaaaga aactgggcga gggatgtggg gtgggcttgc ggagacagga 388
20
gggctgggga gactcgctga gggattcgct cgaggctgag gctggaggga tgtggtggca 388
80
gtggggtcgg gggaacagca ccccagggag aagccagggt ccatggaagt ctgaggcaaa 389
40
aatgtgttgg ctgaggtagg ctccaaagga ctggctgggc tagtgtgcac agcccggatg 390
00
acccagagac caggccagga gcactgcagt gggtgtgtgg agagggttgg gtgggggctg 390
60
catggacagt gtgcatggtt gagaagggag gactccttga ctgcggtcat ccaggaaaag 391
20
acggtgggtt ttgggggggag aattcagagt ccctttgaag gttttaagct tgagctgtat 391
80
taggaaaggt gtgtattagg gaagtatcgg gcttttgaaa cagggacacc cgtgctggat 392
40
gaaggagatg gttgcaccgt ggatc 392
65

## Description

### TECHNICAL FIELD

The present invention relates to a novel human-derived peptide, partial peptides thereof, and DNAs encoding the same. In particular, the present invention relates to a novel peptide having an analgesic peptide-like structure.

### BACKGROUND ART

A number of analgesic peptides having a common amino acid sequence of Tyr-Gly-Gly-Phe-Met/Leu have been isolated and identified to date ever since Met-enkephalin and Leu-enkephalin were isolated in 1975 as analgesic peptides. These peptides have been identified to be partial structures of prepro-opiomelanocortin (Nature, 297, 335-339 (1982)), prepro-enkephalin A, or prepro-enkephalin B (Nature, 297, 431-434 (1982); Nature, 306, 611-614 (1983)). These peptides bind to specific receptors (opiate receptors) on cell membranes thereby to transmit their information to cells. Most of such analgesic peptides have been hitherto isolated from tissue extracts or the like, based on their physiological activities to determine their structures. Recently, receptors have also been utilized to isolate a variety of physiologically active substances or hormones from tissue extracts or the like. For example, nociceptin is known, which is identified as an endogenous ligand to orphan receptor ORL-1. Nociceptin (FGGFTGARKSARKLANQ) is a peptide excised from the nociceptin precursor, and is also considered to be an analgesic peptide.

On the other hand, rapid progress of the genome and cDNA sequence analysis in recent years has enabled to make access to enormous DNA information. It is assumed that these DNAs would include those encoding physiologically active substances hitherto unknown. However, even if an attempt is made to find an unknown physiologically active substance from the genomic DNA sequences or Expressed Sequence Tag (EST), a similar sequence is found also in DNA sequences for genes or non-translational regions of totally unrelated proteins. Further due to a possibility that they might be pseudo genes, it was difficult to find which substance was truly physiologically active in these substances.

As receptors for analgesic peptides, four receptors including three receptors of δ, κ and μ and nociceptin receptor ORL-1 are known so far. These four receptors are all seven-transmembrane receptors and their intracellular signal is known to be associated with reduction of cAMP. It is also known that many analgesic peptides excised from the precursors of four analgesic peptides are cross-reactive with these receptors.

On the other hand, various reports have been made on the physiological activities of these analgesic peptides. First, the analgesic peptides are known to have an analgesic activity as a sensory function. It is also known that they are involved in akinesia (catatonic condition), loss of corneal reflex, eating behavior, increased grooming, accelerated learning effect and suppressed induction of sex behaviors in motor functions and behaviors. In addition, increased prolactin secretion, increased GH secretion, prevention of LH secretion, prevention of TSH secretion, regulation of ACTH secretion, regulation of vasopressin secretion, increased insulin secretion, potentiation of MSH action, etc. are known in endocrine functions. Moreover, it is reported in autonomic functions that analgesic peptides are associated with body temperature control, blood pressure control, respiratory control, gastric secretion, stomach motility, intestinal motility, sleep, etc.

As above, many crucial and physiological actions have been reported with respect to analgesic peptides. However, any analgesic peptide is unknown in mammals, except for those identified as a partial structure of prepro-opiomelanocortin, prepro-enkephalin A or prepro-enkephalin B, or nociceptin identified as an endogenous ligand to orphan receptor ORL-1.

Therefore, it has been desired to find an unknown analgesic peptide derived from human and using the analgesic peptide develop a drug comprising a novel physiologically active substance for the prevention, treatment, diagnosis of diseases.

### DISCLOSURE OF THE INVENTION

The present inventors intensively studied and finally succeeded in cloning a cDNA having a novel nucleic acid sequence by RT-PCR using human testis poly $(A)^+$ RNA as a template and prepared primers. Further, the present inventors found that the polypeptide encoded by the obtained cDNA was a precursor of a useful analgesic peptide. Based on this finding, the present inventors completed the present invention after further investigation.

Thus, the present invention provides:

(1) A protein or a salt thereof comprising the same or substantially the same amino acid sequence as that shown by SEQ ID NO: 1;
(2) A partial peptide of the protein described in item (1), an amide, an ester or a salt thereof;

(3) A DNA comprising a DNA having a nucleic acid sequence encoding the protein described in item (1) or the partial peptide described in item (2), excluding the DNA having the nucleic acid sequence of GenBank Accession Number AC002107 (SEQ ID NO: 5);
(4) The DNA described in item (3) having the nucleic acid sequence shown by SEQ ID NO: 2;
(5) A recombinant vector comprising the DNA described in item (4);
(6) A transformant, which is transformed with the recombinant vector described in item (5);
(7) A method of producing the protein or a salt thereof described in item (1) or the partial peptide, an amide, an ester or a salt thereof described in item (2), which comprises culturing the transformant described in item (6) to produce and accumulate the protein described in item (1) or the partial peptide described in item (2); and recovering it;
(8) A pharmaceutical composition comprising the protein or a salt thereof described in item (1), or the partial peptide, an amide, an ester or a salt thereof described in item (2);
(9) A pharmaceutical composition comprising the DNA described in item (3);
(10) The pharmaceutical composition described in item (8) or (9), which has the analgesic action;
(11) An antibody to the protein or a salt thereof described in item (1), or the partial peptide, an amide, an ester or a salt thereof described in item (2);
(12) A method of screening a receptor agonist or antagonist, which comprises using the protein or a salt thereof described in item (1), or the partial peptide, an amide, an ester or a salt thereof described in item (2);
(13) A kit for screening a receptor agonist or antagonist, which comprises the protein or a salt thereof described in item (1), or the partial peptide, an amide, an ester or a salt thereof described in item (2);
(14) The receptor agonist or antagonist, which is obtained using the screening method described in item (12) or the screening kit described in item (13);
(15) A pharmaceutical composition comprising the receptor agonist or antagonist described in item (14);
(16) The pharmaceutical composition described in item (15), which has the analgesic action;
(17) A method of relieving pain in a mammal, which comprises administering to a mammal an effective amount of the receptor agonist described in item (14);
(18) Use of the receptor agonist described in item (14) for producing an analgesic agent;
(19) A method of relieving pain in a mammal, which comprises administering to a mammal an effective amount of the protein or a salt thereof described in item (1), or the partial peptide, an amide, an ester or a salt thereof described in item (2);
(20) Use of the protein or a salt thereof described in item (1), or the partial peptide, an amide, an ester or a salt thereof described in item (2) for producing an analgesic agent;
(21) A method of relieving pain in a mammal, which comprises administering to a mammal an effective amount of the DNA described in item (3);
(22) Use of the DNA described in item (3) for producing an analgesic agent.

Further, the present invention provides:

(23) The screening method described in item (12), which comprises measuring and comparing the binding amount of the protein or a salt thereof described in item (1), or the partial peptide, an amide, an ester or a salt thereof described in item (2) with a receptor or a partial peptide thereof (i) when the protein or a salt thereof described in item (1), or the partial peptide, an amide, an ester or a salt thereof described in item (2) is brought in contact with said receptor or partial peptide thereof; and (ii) when the protein or a salt thereof described in item (1), or the partial peptide, an amide, an ester or a salt thereof described in item (2) and a test compound are brought in contact with said receptor or partial peptide thereof;
(24) The screening method described in item (12), which comprises measuring and comparing the binding amount of the protein or a salt thereof described in item (1), or the partial peptide, an amide, an ester or a salt thereof described in item (2) with a cell containing a receptor or a cell membrane fraction thereof (i) when the protein or a salt thereof described in item (1), or the partial peptide, an amide, an ester or a salt thereof described in item (2) is brought in contact with said cell containing a receptor or said cell membrane fraction thereof; and (ii) when the protein or a salt thereof described in item (1), or the partial peptide, an amide, an ester or a salt thereof described in item (2) and a test compound are brought in contact with said cell containing a receptor or said cell membrane fraction thereof;
(25) The screening method described in item (12), which comprises measuring and comparing an activity in a cell containing a receptor, such as a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.) (i) when the protein or a salt thereof described in item (1), or the partial peptide, an amide, an ester or a salt thereof described in item (2) is brought in contact with said cell containing a receptor; and (ii) when the protein or a salt thereof described in item (1), or the partial peptide, an amide, an ester or a salt thereof described in item (2) and a test compound are brought in contact with said cell containing a receptor;

(26) A pharmaceutical composition comprising the receptor agonist which is obtained using the screening method described in any of items (12) and (23) to (25), or the screening kit described in item (13);

(27) The pharmaceutical composition described in item (26), which is an analgesic agent;

(28) A pharmaceutical composition comprising the receptor antagonist which is obtained using the screening method described in any of items (12) and (23) to (25), or the screening kit described in item (13);

(29) A method of screening a compound or a salt thereof enhancing or inhibiting an intracellular signal transduction after binding of the protein or a salt thereof described in item (1), or the partial peptide, an amide, an ester or a salt thereof described in item (2), which comprises using the protein or a salt thereof described in item (1), or the partial peptide, an amide, an ester or a salt thereof described in item (2);

(30) The screening method described in item (29), which comprises measuring and comparing the intracellular signal transduction after binding of the protein or a salt thereof described in item (1), or the partial peptide, an amide, an ester or a salt thereof described in item (2) (i) when the protein or a salt thereof described in item (1), or the partial peptide, an amide, an ester or a salt thereof described in item (2) is brought in contact with a cell containing a receptor; and (ii) when the protein or a salt thereof described in item (1), or the partial peptide, an amide, an ester or a salt thereof described in item (2) and a test compound are brought in contact with a cell containing a receptor;

(31) A kit for screening a compound or a salt thereof enhancing or inhibiting an intracellular signal transduction after binding of the protein or a salt thereof described in item (1), or the partial peptide, an amide, an ester or a salt thereof described in item (2), which comprises the protein or a salt thereof described in item (1), or the partial peptide, an

(32) A compound or a salt thereof enhancing or inhibiting an intracellular signal transduction after binding of the protein or a salt thereof described in item (1) or the partial peptide, an amide, an ester or a salt thereof described in item (2), which is obtained using the screening method described in item (29) or (30) or the screening kit described in item (31).

**BRIEF DESCRIPTION OF DRAWINGS**

Fig. 1 shows the nucleic acid sequence of the cDNA encoding the analgesic peptide-like protein of the present invention, obtained in Example 1.

Fig. 2 shows the amino acid sequence of the analgesic peptide-like protein of the present invention, obtained in Example 1.

Fig. 3 shows comparison of the novel analgesic peptide-like protein of the present invention to other known analgesic peptides in terms of amino acid sequence. In this figure, the "novel analgesic peptide" consists of the partial peptide of the 50th residue (Tyr) through the 73rd residue (Lys) of the amino acid sequence shown by SEQ ID NO: 1.

**BEST MODES FOR CARRYING OUT THE INVENTION**

The protein of the present invention is a protein having the same or substantially the same amino acid sequence as that shown by SEQ ID NO: 1 (preferably, an analgesic peptide-like protein).

The protein of the present invention may be derived from any cells (e.g., retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine, intestine duodenum), blood vessel, heart, thymus, spleen, submandibular gland, peripheral blood, peripheral blood cells, prostate, testis, ovary, placenta, uterus, bone, joint, skeletal muscle, etc. from human and other warm-blooded animals (e.g., guinea pigs, rats, mice, rabbits, swine, sheep, bovine, monkeys, etc.). The protein may also be synthesized.

"Substantially the same amino acid sequence as that shown by SEQ ID NO: 1" includes, for example, amino acid sequences having about 40% or more, preferably about 60% or more, more preferably about 80% or more, further preferably about 90% or more, most preferably about 95% or more homology to the amino acid sequence shown by SEQ ID NO: 1.

The protein of the present invention comprising substantially the same amino acid sequence as that shown by SEQ ID NO: 1 is preferably, for example, a protein having substantially the same amino acid sequence as that shown by SEQ ID NO: 1 and also having substantially the same activity with that of a protein comprising the amino acid sequence shown by SEQ ID NO: 1.

Hereinafter in the specification, "a protein comprising the same or substantially the same amino acid sequence as that shown by SEQ ID NO: 1" or "a protein or a salt thereof comprising the same or substantially the same amino acid sequence as that shown by SEQ ID NO: 1" may be referred to simply as "the protein of the present invention".

The activity in the term "substantially the same activity" includes a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.), analgesic activity (action), and the like. The term "substantially the same activity" means qualitative equivalence. Therefore, it is preferable that the proteins have an equivalent level of a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.), analgesic activity (action), and the like (for example, within about 0.5 to about 2-fold), but quantitative factors such as activity levels and molecular weights of the proteins may be different.

Further, the protein of the present invention include so-called muteins thereof, for example, proteins comprising (i) an amino acid sequence having deletion of one or more (e.g. 1 to about 20, preferably 1 to about 9, more preferably several (1 or 2)) amino acids in the amino acid sequence shown by SEQ ID NO: 1, (ii) an amino acid sequence having addition of one or more (e.g. 1 to about 20, preferably 1 to about 9, more preferably several (1 or 2)) amino acids to the amino acid sequence shown by SEQ ID NO: 1, (iii) an amino acid sequence having substitution of one or more (e.g. 1 to about 20, preferably 1 to about 9, more preferably several (1 or 2)) amino acids by other amino acids in an amino acid sequence shown by SEQ ID NO: 1.

If the amino acid sequence has the above-mentioned deletion or substitution, the position of amino acid residue to be deleted or substituted is not specifically limited.

In the present specification, proteins are represented in accordance with the conventional way of describing peptides, placing the N-terminus (amino terminus) on the left side and the C-terminus (carboxyl terminus) on the right side. In the protein of the present invention including the protein comprising the amino acid sequence shown by SEQ ID NO: 1, the C-terminus is usually in the form of a carboxyl group (-COOH) or a carboxylate ($-COO^-$), and also may be in the form of an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$-alkyl group, e.g., benzyl, phenethyl, etc., or an α-naphthyl-$C_{1-2}$-alkyl group such as α-naphthylmethyl, etc.; and the like. In addition, pivaloyloxymethyl or the like, which is used widely as an ester for oral administration, may also be used.

When the protein of the present invention contains a carboxyl group (or a carboxylate) at a position other than the C-terminus, it may be amidated or esterified. Such an amide or ester is also included within the protein of the present invention. The ester in this case may be the same as described above with respect to the ester of C-terminus.

Furthermore, the protein of the present invention includes variants of the above-mentioned proteins, wherein the amino group at the N-terminal methionine residue of the protein is protected with a protecting group (e.g. $C_{1-6}$ acyl group such as formyl group, acetyl group, etc.); those wherein a glutamyl group, which is formed due to cleavage at the N-terminal side in vivo, is pyroglutaminated; those wherein a substituent (e.g. -OH, -SH, amino group, imidazole group, indole group, guanidino group, etc.) on the side chain of an amino acid in the molecule is protected with a suitable protecting group (e.g. $C_{1-6}$ acyl group such as formyl group, acetyl group, etc.), or conjugated proteins such as glycoproteins bound to sugar chains.

A partial peptide of the protein of the present invention (hereinafter, a partial peptide of a protein comprising the same or substantially the same amino acid sequence as that shown by SEQ ID NO: 1, an amide, an ester or a salt thereof may be generically referred to as the partial peptide of the protein of the present invention) includes any peptide having the same activity as that of the protein of the present invention, for example, a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.), analgesic activity (action), and the like.

Specifically used are a partial peptide having the 19th residue (Asp) to 73rd residue (Lys) of the amino acid sequence shown by SEQ ID NO: 1, a partial peptide having the 50th residue (Tyr) to 73rd residue (Lys) of the amino acid sequence shown by SEQ ID NO: 1, and the like.

Furthermore, preferred is the partial peptide of the present invention having substantially the same amino acid sequence as that shown by SEQ ID NO: 1 and substantially the same activity as that of a peptide comprising the amino acid sequence shown by SEQ ID NO: 1.

The activity in the term "substantially the same activity" includes a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.), analgesic activity (action), and the like. The term "substantially the same activity" means qualitative equivalence. Therefore, it is preferable that the proteins have an equivalent level of a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.), analgesic activity (action), and the like (for example, within about 0.5 to about 2-fold), but quantitative factors such as activity levels and molecular weights of the proteins may be different.

The partial peptide of the present invention also includes one having (antagonistic) inhibitory activity against the protein of the present invention, that is, one capable of inhibiting the activity of the protein of the present invention.

Furthermore, the partial peptide of the present invention includes partial peptides of proteins having at least about 40% homology, preferably at least about 60% homology, more preferably at least about 80% homology, much more preferably at least about 90% homology, and most preferably at least about 95% homology to the amino acid sequence shown by SEQ ID NO: 1. More specifically, the partial peptide of the present invention include partial peptides of proteins comprising an amino acid sequence having deletion of one or more (e.g. 1 to about 20, preferably 1 to about 9, more preferably several (1 or 2)) amino acids in the amino acid sequence shown by SEQ ID NO: 1; an amino acid sequence having addition of one or more (e.g. 1 to about 20, preferably 1 to about 9, more preferably several (1 or 2)) amino acids to the amino acid sequence shown by SEQ ID NO: 1; or an amino acid sequence having substitution of one or more (e.g. 1 to about 20, preferably 1 to about 9, more preferably several (1 or 2)) amino acids by other amino acids in an amino acid sequence shown by SEQ ID NO: 1.

In the partial peptide of the present invention, the C-terminus is normally a carboxyl group (-COOH) or carboxylate ($-COO^-$), but the C-terminus may be in the form of an amide ($-CONH_2$) or an ester (-COOR), as described in the protein of the present invention.

Furthermore, the partial peptide of the present invention also includes variants of the above-mentioned peptides, wherein the amino group at the N-terminal methionine residue of the protein is protected with a protecting group (e.g. $C_{1-6}$ acyl group such as formyl group, acetyl group, etc.); those wherein a glutamyl group, which is formed due to cleavage at the N-terminal side in vivo, is pyroglutaminated; those wherein a substituent (e.g. -OH, -SH, amino group, imidazole group, indole group, guanidino group, etc.) on the side chain of an amino acid in the molecule is protected with a suitable protecting group (e.g. $C_{1-6}$ acyl group such as formyl group, acetyl group, etc.), or conjugated peptides such as glycopeptides bound to sugar chains.

For a salt of the protein or the partial peptide of the present invention, especially, a physiologically acceptable acid addition salt is preferred. Examples of the salt include a salt with an inorganic acid (e.g., hydrochloric acid, phosphoric acid, hydrobromic acid, sulfuric acid) or with an organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The protein of the present invention or a salt thereof may be produced by a known purification method from cells, tissues or blood plasma of a human or a warm-blooded animal as described above, or by culturing a transformant comprising a DNA encoding the protein as later described. Furthermore, the protein or its salt may also be produced by a protein synthetic method as described below or a modified method thereof.

When the protein or its salt is produced from cells, tissues or blood plasma of a human or a warm-blooded animal, it is purified and isolated from a supernatant of homogenized tissues or cells, or from blood plasma using ammonium sulfate precipitation, ethanol precipitation, acid extraction, and a combination of chromatography techniques such as ion exchange chromatography, hydrophobic chromatography, hydroxyapatite chromatography, reverse phase chromatography, lectin column chromatography, gel filtration chromatography.

To synthesize the protein of the present invention, a partial peptide, a salt, or an amide thereof, commercially available resins that are used for protein synthesis may be usually used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the protein is cut out from the resin and at the same time, the protecting groups are removed. Then, intramolecular disulfide bond-forming reaction is performed in a highly diluted solution to obtain the objective protein, a partial peptide or an amide thereof.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by e.g. alkyl esters (e.g. esters of methyl, ethyl, propyl, butyl, t-butyl, cyclopentyl, cyclohexyl, cycloheptyl, cyclooctyl, 2-adamantyl, etc.), benzyl ester, 4-nitrobenzyl ester, 4-methoxybenzyl ester, 4-chlorobenzyl ester" benzhydryl ester, phenacyl ester, benzyloxycarbonyl hydrazide, t-butoxycarbonyl hydrazide, trityl hydrazide, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl group in the starting material include the corresponding acid anhydride, azide, activated ester (ester with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). Examples of the activated amino group in the starting material include a phosphoric amide.

To eliminate (remove) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group used as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining an amide of the protein, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives an amide of the desired protein.

To prepare the esterified protein; for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide of the protein of the present invention or a salt thereof can be produced by publicly known methods for peptide synthesis, or by cleaving the protein of the present invention with an appropriate peptidase. For the methods for peptide synthesis, for example, either solid phase synthesis or liquid phase synthesis may be used. That is, a partial peptide or an amino acid that can construct the protein of the present invention are condensed with the remaining part. Where the product contains protecting groups, these protecting groups are removed to give the desired peptide. Publicly known methods for condensation and elimination of the protecting groups are described in 1) - 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

The DNA encoding the protein of the present invention includes any DNA comprising a nucleotide sequence encoding the protein of the present invention described above, and may be derived from any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using an mRNA fraction prepared from the cells and tissues described above.

Specifically, the DNA encoding the protein of the present invention having the amino acid sequence shown by SEQ ID NO: 1 may be (i) a DNA having the nucleic acid sequence shown by SEQ ID NO: 2, or (ii) a DNA which is hybridizable to the DNA having the nucleic acid sequence shown by SEQ ID NO: 2 and encodes a protein having substantially the same activity as that of the protein having the amino acid sequence shown by SEQ ID NO: 1, for example, a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.), angiogenesis activity (action), cell proliferation, cell migration or cell differentiation activity (action), etc.

Examples of the DNA hybridizable to the nucleic acid sequence shown by SEQ ID NO: 2 include a DNA containing a nucleic acid sequence having at least about 40% homology, preferably at least about 60% homology, more preferably at least about 80% homology, much more preferably at least about 90% homology, and most preferably at least about 95% homology to the nucleic acid sequence shown by SEQ ID NO: 2.

The hybridization can be carried out by publicly known methods or by modifications thereof, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, the DNA encoding the protein comprising the amino acid sequence shown by SEQ ID NO: 1 includes the DNA having the nucleic acid sequence shown by SEQ ID NO: 2.

The DNA encoding the partial peptide of the present invention may be any DNA comprising the nucleotide sequence encoding the partial peptide of the present invention as described above.

Specifically, a DNA encoding a partial peptide having the 19th residue (Asp) to 73rd residue (Lys) of the amino acid sequence shown by SEQ ID NO: 1 includes a DNA having the 55th to 219th of the nucleotide sequence shown by SEQ ID NO: 2; and a DNA encoding a partial peptide having the 50th residue (Tyr) to 73rd residue (Lys) of the amino acid sequence shown by SEQ ID NO: 1 includes a DNA having the 148th to 219th of the nucleotide sequence shown by SEQ ID NO: 2.

For cloning of the DNA that completely encodes the protein of the present invention or a partial peptide thereof (hereinafter sometimes referred to simply as the protein of the present invention), the DNA may be either amplified by PCR using synthetic DNA primers containing a part of the nucleic acid sequence encoding the protein of the present invention. Alternatively, the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd, J. Sambrook et al., Cold Spring Harbor Lab. Press, 1989. The hybridization may also be performed using a commercially available library in accordance with the protocol described in the attached instruction.

The cloned DNA encoding the protein of the present invention or a partial peptide thereof can be used depending upon purpose, as it is or if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the DNA encoding the protein of the present invention or a partial peptide thereof can be produced, for example, by (a) excising the desired DNA fragment from the DNA encoding the protein of the present invention, and then (b) ligating the DNA fragment downstream of a promoter in an appropriate expression vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ-phage, etc., animal viruses such as retrovirus, vaccinia virus, baculovirus, etc. as well as pA1-11, pXTl, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc. Among them, CMV promoter or SRα promoter is preferably used.

When the host is bacteria of the genus Escherichia, preferred examples of the promoter include trp promoter, lac promoter, recA promoter, $\lambda P_L$ promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker in CHO ($dhfr^-$) cells, selection can also be made on a thymidine-free medium.

If necessary and desired, a signal sequence suitable for a host is added to the N-terminus of the protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

By introducing the vector containing the DNA encoding the protein of the present invention thus constructed into a cell, a transformant can be produced.

Examples of the host, which may be employed, are bacteria belonging to the genus Escherichia, bacteria belonging to the genus Bacillus, yeast, insect cells, insects and animal cells, etc.

Examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vitro, 13, 213-217 (1977).

Examples of insects include a larva of Bombyx mori (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO ($dhfr^-$) cell), L cells, myeloma cells, human FL cells, 293 cells, C127 cells, BALB3T3 cells, Sp-2/O cells, etc. Among those, CHO cells, CHO ($dhfr^-$) cells, 293 cells are preferred.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991).

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995) (published by Shujunsha).

The method of introducing the expression vector into the cell includes, for example, calcium phosphate method (Graham, F.L. and van der Eb, A.J. Virology, 52, 456-467 (1973)), DEAE-dextran method (Sompayrac, L.M. and Danna, K.J., Proc. Natl. Acad. Sci. U.S.A., 78, 7575-7578, 1981), lipofection method (Malone, R.W., Proc. Natl. Acad. Sci. U.S.A., 86, 6077-6081, 1989), electroporation method (Nuemann, E. et al. Embo J., 1, 841-845 (1982)), etc.

Thus, a transformant, which is transformed with the expression vector containing the DNA encoding the protein of the present invention, can be obtained.

Furthermore, to express the protein of the present invention in a stable manner using animal cells, the animal cell clone can be selected, the chromosome of which the introduced expression vector is incorporated into. To be specific, using the above selection marker as an index, a transformant can be selected. From these animal cells obtained by use of the selection marker, it is possible to obtain a stable animal cell strain expressing highly the protein of the present invention by repeating the clonal selection.

Moreover, when using dhfr gene as a selection marker, the cells are cultured in gradually increased concentrations of MTX, and an MTX-resistant cell strain is selected. In this way, it is possible to obtain an animal cell strain with higher expression by amplifying the DNA encoding the protein of the present invention as well as dhfr gene in the cell.

The protein of the present invention, a partial peptide or a salt thereof can be produced by cultivating the above-mentioned transformant under condition allowing expression of the DNA encoding the protein of the present invention or a partial peptide thereof; and producing and accumulating the protein of the present invention or a partial peptide thereof.

When the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

When the bacteria belonging to the genus Escherichia are used as the host, the transformant is usually cultivated at about 15°C to about 43°C for about 3 hours to about 24 hours. If necessary and desired, the culture may be aerated or agitated.

When the bacteria belonging to the genus Bacillus are used as the host, the transformant is cultivated generally at about 30°C to about 40°C for about 6 hours to about 24 hours. If necessary and desired, the culture can be aerated or agitated.

When yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

When insect cells are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

When animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 72 hours and, if necessary and desired, the culture can be aerated or agitated.

When using CHO ($dhfr^-$) cells and dhfr gene as a selection marker, a thymidine-free DMEM medium containing dialyzed fetal bovine serum is preferably used.

The protein of the present invention can be separated and purified from the culture described above by the following procedures.

When the protein of the present invention is extracted from the cultured bacteria or cells, after cultivation, the bacteria or cells are collected by a publicly known method and suspended in an appropriate buffer. The bacteria or cells are then disrupted by publicly known methods such as ultrasonication, a treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100™.

When the protein is secreted into the culture medium, after completion of the cultivation, the supernatant can be separated from the bacteria or cells to collect the supernatant by a publicly known method. The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, it can be converted into a salt by publicly known methods or modifications thereof. On the other hand, when the protein is obtained in the form of a salt, it can be converted into the free form or a different salt by publicly known methods or modifications thereof.

The protein of the present invention, produced by the recombinant, can be treated, before or after the purification, with an appropriate protein-modifying enzyme so that the protein can be appropriately modified or can be partially deleted. Examples of the protein-modifying enzyme include trypsin, chymotrypsin, arginyl endopeptidase, protein kinase, glycosidase or the like.

The presence of the thus produced protein of the present invention can be determined by an enzyme immunoassay using a specific antibody, or the like.

Antibodies to the protein of the present invention, a partial peptide, or a salt thereof may be any of polyclonal antibodies and monoclonal antibodies, which are capable of recognizing the protein of the present invention, a partial peptide, or a salt thereof (hereinafter sometimes referred to as the protein of the present invention).

The antibodies to the protein of the present invention (hereinafter sometimes referred to as the antibody of the present invention) may be produced according to a publicly known method for producing antibodies or antisera, using as an antigen the protein of the present invention.

[Preparation of monoclonal antibody]

(a) Preparation of monoclonal antibody-producing cells

The protein of the present invention is administered to warm-blooded animals either alone or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable warm-blooded animals are monkeys, rabbits, dogs, guinea pigs, mice, rats, sheep, goats and chicken, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the protein, which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be manufactured by publicly known methods or modifications thereof. For example, a complex of immunogen (a protein antigen) and a carrier protein is prepared, and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of an immunogen and a carrier protein used to immunize a warm-blooded animal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either alone or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of a warm-blooded animal immunized by the method described above.

The polyclonal antibody titer in antiserum can be assayed by the same procedure as that for the determination of serum antibody titer described above. The separation and purification of the polyclonal antibody can be carried out according to the method for the separation and purification of immunoglobulins, which is applied to the separation and purification of monoclonal antibodies as described above.

An antisense DNA having a nucleic acid sequence complementary to a DNA or an mRNA encoding the protein of the present invention or a partial peptide thereof may be an oligonucleotide or a derivative thereof, which has a nucleic acid sequence complementary to the whole or a part of a DNA sequence or an mRNA sequence encoding the protein of the present invention or a partial peptide thereof, and has an activity to inhibit the expression of said protein or partial peptide.

Examples of said complementary nucleic acid sequence include nucleic acid sequences having at least about 40%, preferably at least about 60%, more preferably at least about 80% and much more preferably at least about 90% homology to the whole or a part of a DNA sequence or an mRNA sequence encoding the protein of the present invention or a partial peptide thereof. Particularly preferred are antisense DNAs having at least about 40%, preferably at least about 60%, more preferably at least about 80% and much more preferably at least about 90% homology to a part of the whole nucleic acid sequence of the DNA or mRNA of the present invention, the part which encodes a N-terminal region of the protein of the present invention. These antisense DNAs can be produced a well-known DNA synthesizer, etc.

The protein of the present invention, a partial peptide thereof, or a salt thereof has an activity, such as a receptor-mediated cell-stimulating activity (e.g. arachidonic acid release, acetylcholine release, intracellular $Ca^{2+}$ release, intracellular cAMP production, intracellular cGMP production, inositol phosphate production, cell membrane potential change, phosphorylation of intracellular proteins, pH decrease, etc.), analgesic activity (action), and the like. Therefore, the protein of the present invention, a partial peptide thereof, or a salt thereof can be used for various applications.

Hereinafter, use of the protein of the present invention, a partial peptide or a salt thereof (hereinafter sometimes referred to as the protein of the present invention), use of the DNA encoding the protein of the present invention (hereinafter sometimes referred to as the DNA of the present invention), use of the antibody to the protein of the present invention (hereinafter sometimes referred to as the antibody of the present invention), and use of the antisense DNA are specifically described.

**<1> A prophylactic and/or therapeutic agent for various diseases**

The protein of the present invention or the DNA of the present invention is useful as a pharmaceutical such as an analgesic.

When the protein of the present invention or the DNA of the present invention is used as a pharmaceutical, it can be used orally, for example, in the form of a tablet which may be sugar-coated if necessary, a capsule, an elixir, an microcapsule, etc., or parenterally in the form of an injectable preparation such as a sterile solution and a suspension with water or other pharmaceutically acceptable liquids. These preparations can be produced by mixing the protein of the present invention or the DNA of the present invention with a physiologically acceptable carrier, a flavoring agent, an excipient, a vehicle, an antiseptic agent, a stabilizer, a binder, etc. in a generally accepted unit dosage form required for pharmaceutical compositions. A dose of the effective component in these preparations is adjusted appropriately within the specified range.

When the DNA of the present invention is used, the DNA can be administered by itself. Alternatively, the DNA, which is inserted into an appropriate vector such as retrovirus vector, adenovirus vector, adenovirus-associated virus vector, etc., can be administered in a conventional manner.

Additives miscible in a tablet, a capsule, etc. include a binder such as gelatin, corn starch, tragacanth and gum arabic; an excipient such as crystalline cellulose; a swelling agent such as corn starch, gelatin and alginic acid; a lubricant such as magnesium stearate; a sweetening agent such as sucrose, lactose and saccharin; and a flavoring agent such as peppermint, akamono oil and cherry. When the unit dosage is in the form of a capsule, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include a physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol. The composition may further contain a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid injection is normally filled in an appropriate ampoule.

Since the thus obtained pharmaceutical composition is safe and low toxic, the preparation can be administered to a human or a warm-blooded animal (e.g. a mammal such as a human, rat, mouse, guinea pig, rabbit, bird, sheep, swine, bovine, horse, cat, dog, monkey).

The dose of the protein or the DNA varies depending on conditions to be treated, etc. In oral administration to a normal adult (60 kg body weight), the daily dose of the effective component is generally about 0.1 mg to about 100 mg, preferably about 1.0 to about 50 mg, and more preferably about 1.0 to about 20 mg. In parenteral administration, the single dose also varies depending on subject to be administered, target organ, conditions, routes for administration, etc. For example, it is advantageous to administer to a normal adult (60 kg body weight) the active ingredient intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

**<2> A genetically diagnostic agent**

By using the DNA of the present invention as a probe, an aberration (gene aberration) of the DNA encoding the protein of the present invention or its partial peptide can be detected in a warm-blooded animal (e.g., human, rat, mouse, guinea pig, rabbit, sheep, swine, bovine, horse, cat, dog, monkey, etc.). Therefore, the DNA of the present invention is useful as a gene diagnostic agent for detecting diseases involving the protein of the present invention.

The genetic diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

**<3> Quantification of the protein of the present invention, a partial peptide, or a salt thereof**

The antibodies of the present invention are capable of specifically recognizing the protein of the present invention. Therefore, the antibodies can be used to quantify the protein of the present invention in a test liquid, especially for quantification by the sandwich immunoassay.

Thus, the present invention provides the following quantification methods:

(i) a method of quantifying the protein of the present invention in a test liquid, which comprises competitively reacting the antibody of the present invention with the test liquid and the labeled protein, and measuring the ratio of the labeled protein bound to the antibody; and
(ii) a method of quantifying the protein of the present invention in a test liquid, which comprises reacting the test liquid with the antibody of the present invention immobilized on a carrier and the labeled antibody of the present invention simultaneously or sequentially, and measuring the activity of the label on the immobilized carrier.

In (ii) described above, it is preferred that one antibody recognizes the N-terminal region of the protein of the present invention, and the other antibody reacts with the C-terminal region of the protein of the present invention.

Using monoclonal antibodies to the protein of the present invention (hereinafter sometimes referred to as the monoclonal antibodies of the present invention), the protein of the present invention can be quantified, and also detected by tissue staining. For this purpose, an antibody molecule itself may be used, or $F(ab')_2$, Fab' or Fab fractions of the antibody molecule may also be used.

Types of quantification methods using antibodies to the protein of the present invention are not particularly limited. Any assay methods can be used if the amount of antibody, antigen, or antibody-antigen complex corresponding to the amount of antigen (e.g., the amount of the protein) in the test liquid can be detected by chemical or physical means and the amount of the antigen can be calculated from a standard curve prepared from standard solutions containing known amounts of the antigen. For example, a nephrometry, a competitive method, an immunometric method, and a sandwich method are appropriately used, with the sandwich method described below being most preferable in terms of sensitivity and specificity.

As a labeling agent used for measurement with a labeled substance, there are employed, for example, radioisotopes, enzymes, fluorescent substances, luminescent substances, etc. For the radioisotope, for example, [$^{125}$I], [$^{131}$I], [$^{3}$H] and [$^{14}$C] are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used are fluorescamine and fluorescein isothiocyanate. For the luminescent substance, for example, luminol, luminol derivatives, luciferin, and lucigenin are used. Furthermore, the biotin-avidin system may be used for binding of antibody or antigen to the label.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose; synthetic resin such as polystyrene, polyacrylamide, silicon; or glass are used.

In the sandwich method, the immobilized monoclonal antibody of the present invention is reacted with a test liquid (primary reaction), then with the labeled monoclonal antibody of the present invention (secondary reaction), and the activity of the label on the immobilizing carrier is measured, whereby the amount of the protein of the present invention in the test liquid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the quantification of the protein of the present invention by the sandwich method, antibodies that bind to different sites of the protein are preferably used as the monoclonal antibodies of the present invention for the primary and secondary reactions. This means, in respect to the antibodies used for the primary and secondary reactions, if the antibody used in the secondary reaction recognizes the C-terminal region of the protein, the antibody used in the primary reaction preferably recognize a region other than the C-terminal region, for example, the N-terminal region.

The monoclonal antibodies of the present invention can be used for the assay systems other than the sandwich method, for example, the competitive method, the immunometric method, and the nephrometry.

In the competitive method, antigen in a test liquid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test liquid is quantified.

This reaction method includes a liquid phase method using a soluble antibody as an antibody, polyethylene glycol for B/F separation and a secondary antibody to the soluble antibody; and an immobilized method either using an immobilized antibody as the primary antibody, or using a soluble antibody as the primary antibody and immobilized antibody as the secondary antibody.

In the immunometric method, after antigen in a test liquid and immobilized antigen are competitively reacted with a given amount of labeled antibody, the immobilized phase is separated from the liquid phase. Alternatively, after antigen in a test liquid and an excess amount of labeled antibody are reacted, and an immobilized antigen is added to bind the unreacted labeled antibody with the immobilized phase, the immobilized phase is separated from the liquid phase. Then, the amount of the label in either phase is measured to quantify the antigen in the test liquid.

In the nephrometry, an insoluble precipitate produced after the antigen-antibody reaction in gel or solution is quantified. When the amount of antigen in the test liquid is small and only a small amount of precipitate is obtained, laser nephrometry using scattering of laser is advantageously employed.

For applying these immunological methods to the quantification method of the present invention, any special conditions or procedures are not required. Systems for quantifying the protein of the present invention are constructed by combining the usual technical consideration in the art to the conventional conditions and procedures. For the details of these general technical means, reference can be made to any reviews and texts.

See, for example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the protein of the present invention can be quantified with high sensitivity using the antibodies of the present invention.

By measuring the concentration of the protein of the present invention using the antibodies of the present invention, it is possible to make diagnosis for diseases involving the protein of the present invention.

The antibodies of the present invention can also be used for specifically detecting the protein of the present invention present in test samples such as body fluids or tissues. The antibodies may also be used for preparation of antibody columns for purification of the protein of the present invention, for detection of the protein of the present invention in each fraction upon purification.

**<4> Screening of a candidate compound for a pharmaceutical**

(A) Method of screening the receptor agonist or antagonist

By constructing the ligand-receptor binding assay system using the protein of the invention and a receptor thereto, a pharmaceutical candidate compound having a similar activity to that of the protein of the invention can be screened, or a pharmaceutical candidate compound that inhibits the activity of the protein of the invention can be screened. That is, the present invention provides a method of screening the receptor agonist or antagonist using the protein of the invention.

More specifically, the present invention provides:

(1) a method of screening the receptor agonist or antagonist, which comprises making comparison in cases (i) when the protein of the invention is brought in contact with the receptor or its partial peptide and (ii) when the protein of the invention and a test compound are brought in contact with the receptor or its partial peptide; and
(2) a method of screening the receptor agonist or antagonist, which comprises making comparison in cases (i) when the protein of the invention is brought in contact with a cell containing the receptor or its cell membrane fraction and (ii) when the protein of the invention and a test compound are brought in contact with a cell containing the receptor or its cell membrane fraction.

Specifically, the screening method of the present invention is characterized by determining and comparing, for example, the binding amount of the protein of the invention to its receptor or a cell containing the receptor, a receptor-mediated cell stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity in (i) and (ii).

More specifically, the present invention provides:

(1a) a method of screening the receptor agonist or antagonist, which comprises determining and comparing the binding amount of a labeled form of the protein of the invention to the receptor or its partial peptide or a salt thereof, in cases (i) when the labeled form of the protein of the invention is brought in contact with the receptor or its partial peptide and (ii) when the labeled form of the protein of the invention and a test compound are brought in contact with the receptor or its partial peptide;

(2a) a method of screening the receptor agonist or antagonist, which comprises determining and comparing the binding amount of a labeled form of the protein of the invention to a cell containing the receptor or its cell membrane fraction, in cases (i) when the labeled form of the protein of the invention is brought in contact with a cell containing the receptor or its cell membrane fraction and (ii) when the labeled form of the protein of the invention and a test compound are brought in contact with a cell containing the receptor or its cell membrane fraction; and

(2b) a method of screening the receptor agonist or antagonist which comprises determining and comparing a receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity, in cases (i) when the protein of the invention is brought in contact with a cell containing the receptor and (ii) when the protein of the invention and a test compound are brought in contact with a cell containing the receptor.

In the screening method of (1a) or (2a) described above, a compound that binds to the receptor to inhibit the binding of the protein of the invention to the receptor can be selected as the receptor agonist or antagonist.

In the screening method of (2b) described above, a compound that binds to the receptor to promote a cell-stimulating activity mediated by the receptor (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), or exhibit an analgesic activity or other activity, can be selected as the receptor agonist, while a compound having an activity of suppressing the cell-stimulating activity can be selected as the receptor antagonist.

In the test compounds which are recognized in the screening method (1a) or (2a) described above to have the inhibitory activity against the binding of the protein of the invention to the receptor, a compound having a receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity can be selected as the receptor agonist, whereas a compound having no such an activity can be selected as the receptor antagonist.

Preferably, human-derived or warm-blooded animal-derived analgesic peptide receptors (δ, κ, μ) and the like are used as the receptors for the screening methods of the present invention.

These receptors and receptors to the protein of the invention may be obtained in accordance with known methods for protein purification. The desired receptors may also be obtained by cloning DNAs encoding the receptors in accordance with known genetic engineering procedures and then following the methods of expressing the protein of the invention described above.

A partial peptide obtained by suitably cleaving the full-length receptor can be employed as the partial peptide of the receptor.

Examples of a labeled form of the protein of the invention, which may be employed, include the protein of the invention labeled with [$^{3}H$], [$^{125}I$], [$^{14}C$], [$^{35}S$], etc.

The same cells given as host cells used to express the protein of the invention described above may be employed as the aforesaid cells containing the receptor, which are used for the screening methods of the present invention. Among others, CHO cells, etc. are preferred. The receptor-containing cells may be produced by known methods, e.g., in accordance with the methods for expressing the protein of the invention described above, etc. Cell lines such as CL8 cell line (BONE, 18, 159-169, 1996), OK cell line (AMERICAN JOURNAL OF PHYSIOLOGY, 253, E221-E227, 1987), etc. may also be used as the receptor-containing cells described above.

In the screening methods of the present invention, when the receptor-containing cells are used, the cells may be fixed using glutaraldehyde, formalin, etc. The fixation can be effected by publicly known methods.

The cell membrane fraction of the receptor-containing cell described above refers to a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about I to 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor expressed or the peptide of the invention and membrane components such as cell-derived phospholipids, membrane proteins, etc.

The amount of the receptor in the receptor-containing cell or in the cell membrane fraction is preferably $10^3$ to $10^8$ molecules per cell, more preferably $10^5$ to $10^7$ molecules per cell. As the amount of expression increases, the ligand binding activity per unit of the membrane fraction (specific activity) increases so that not only the highly sensitive screening system can be constructed but also large quantities of samples can be assayed with the same lot.

Examples of the test compounds include proteins, non-proteinaceous compounds, synthetic compounds, fermentation products, cell extracts, vegetable extracts, animal tissue extracts, etc. and these compounds may be novel compounds or publicly known compounds.

In the screening methods of the present invention, the reaction between the protein of the invention and the receptor can be carried out normally at about 37°C for several hours.

Specifically, the screening method of (1a) or (2a) described above can be performed by the following procedures. First, a receptor preparation is prepared by suspending cells containing the receptor of the present invention or cell membrane fractions in a buffer appropriate for screening. Any buffer can be used so long as it does not interfere with the binding between the protein of the invention and the receptor, such buffers including a phosphate buffer or a Tris-HCl buffer, having pH of approximately 4 to 10 (preferably pH of approximately 6 to 8), etc. For the purpose of minimizing non-specific binding, a surfactant such as CHAPS, Tween-80™ (manufactured by Kao-Atlas Inc.), digitonin, deoxycholate, etc. may be added to the buffer. Besides, for the purpose of reducing the degradation of the receptor or ligand by a protease, a protease inhibitor such as PMSF, leupeptin, bacitracin, aprotinin, E-64 (manufactured by Peptide Institute, Inc.), pepstatin, etc. may also be added. On the other hand, when the cells are fixed, the protein of the invention can be bound to the receptor using the cells attached to an incubator, namely, in such a state that the cells are growing, or using the cells fixed with glutaraldehyde or paraformaldehyde.

In this case, a culture medium, Hanks' solution or the like is used as the buffer. Then, a predetermined quantity (in the case of 2,000 Ci/mmol, for example, approximately 10,000 cpm to 1,000,000 cpm) of a labeled form of the protein of the invention (for example, the protein of the invention labeled with [$^{125}$I]) is added to 0.01 to 10 ml of the receptor solution together with a test compound at a concentration of $10^{-4}$ M to $10^{-10}$ M. To find the amount of non-specific binding (NSB), a reaction tube containing a large excess of the protein of the invention in an unlabeled form is also provided. The reaction is carried out at 0°C to 50°C, preferably 4°C to 37°C for 20 minutes to 24 hours, preferably 30 minutes to 3 hours. After completion of the reaction, the reaction mixture is filtrated through a glass fiber filter paper or the like and washed with a suitable volume of the same buffer. The residual radioactivity (e.g., an amount of [$^{125}$I]) remaining on the glass fiber filter paper is then measured by means of a liquid scintillation counter or a γ-counter. For filtration, a manifold or a cell harvester can be employed but the use of a cell harvester is preferred for increased efficiency. With the balance ($B_0$ - NSB) after subtracting the amount of non-specific binding (NSB) from the count ($B_0$) without an antagonistic test compound being taken as 100%, a test compound giving a specific binding value (B-NSB) of not more than 50% of the count ($B_0$-NSB) can be selected as a candidate agonist or antagonist compound.

To perform the screening method (2b) described above, the activities such as the receptor-mediated cell-stimulating activity (for example, arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), the analgesic activity or other activity can be assayed by publicly known methods or a modification thereof.

Specifically, the receptor-containing cells are cultivated on a multi-well plate or the like in the first place. Prior to screening, the medium is replaced with a fresh medium or a suitable non-cytotoxic buffer and, after addition of the test compound etc., the plate is incubated for a predetermined period of time. Then, after the cells are extracted or the supernatant is recovered, products formed are quantified in accordance with the corresponding method. When it is difficult to assay the formation of an indicator for the cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) due to a proteolytic enzyme contained in the cells, an inhibitor to the enzyme may be added prior to the assay. As to an activity to suppress the cAMP production or the like, the inhibitory activity can be assayed against cells with basal production enhanced by forskolin or the like beforehand.

The analgesic activity can be assayed according to publicly known methods.

In the above screening method (2b), when the receptor-containing cell shows an increase in the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an increase in the analgesic activity or other activity by addition of a test compound, the test compound can be selected as a candidate compound of the receptor agonist. On the other hand, when the receptor-containing cell shows a decrease in the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), a decrease in the analgesic activity or other activity by addition of a test compound, the test compound can be selected as a candidate compound of the receptor antagonist.

The screening kit according to the present invention comprises the protein of the present invention and preferably, and further comprises the receptor-containing cell, a cell membrane fraction thereof, or the like.

The following is an example of the screening kit of the present invention.

[Reagents for screening]

(1) Assay buffer and wash buffer

Hanks' Balanced Salt Solution (manufactured by Gibco) supplemented with 0.05% of bovine serum albumin (manufactured by Sigma). The buffers may be sterilized by filtration through a membrane filter with a 0.45 µm pore size and stored at 4°C, or may be prepared at use.

(2) Receptor preparation

CHO cells containing the receptor, etc. to the protein of the present invention are subcultured at 5 x $10^5$ cells/well on a 12-well plate at 37°C under 5% $CO_2$ and 95% air for 2 days.

(3) Preparation of the protein of the invention in a labeled form

The protein of the invention, its partial peptide or a salt thereof is labeled with [$^3$H], [$^{125}$I], [$^{14}$C], [$^{35}$S], etc.

(4) Standard solution of the protein of the invention

The protein of the invention, its partial peptide or a salt thereof is dissolved in PBS containing 0.1% of bovine serum albumin (manufactured by Sigma) to make the volume 0.1 mM and then stored at -20°C.

[Procedures for assay]

(1) The CHO cells containing recombinant receptor, cultivated in a 12-well tissue culture plate, are washed twice with 1 ml of the assay buffer, followed by addition of 490 μl of the assay buffer to each well.
(2) After 5 μl of a test compound solution of $10^{-3}$ to $10^{-10}$ M is added, 5 μl of the protein of the invention of 5 nM in a labeled form is added to the system followed by reacting at room temperature for an hour. In order to find the amount of the non-specific binding, 5 μl of the protein of the invention of $10^{-4}$ M is added to the system in place of the test compound.
(3) The reaction solution is removed, and the plate is washed three times with 1 ml each of the assay buffer. The labeled protein of the invention bound to the cells is dissolved in 0.5 ml of 0.2N NaOH-1% SDS and the solution is mixed with 4 ml of liquid scintillator A (manufactured by Wako Pure Chemical Industries, Ltd.).
(4) Radioactivity is measured with a liquid scintillation counter (manufactured by Beckman) and percent of the maximum binding (PMB) is calculated in accordance with the following equation. When the protein is labeled with [$^{125}$I], the radioactivity can be directly measured with a gamma-ray counter without mixing with the liquid scintillator.

$$PMB = 100 \times (B-NSB)/(B_0 - NSB)$$

wherein:

PMB: percent of the maximum binding
B: value when the sample is added
NSB: non-specific binding
$B_0$: maximum binding

As above, the protein of the invention is useful as a reagent for screening the receptor agonist or antagonist. The compound or its salt, which is obtained using the screening methods or the screening kits of the present invention, inhibits the binding of the protein of the invention to the receptor, and specifically the compound or its salt has an activity such as the receptor-mediated cell stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity (a so-called receptor agonist); or the compound or its salt has no activity such as the receptor-mediated cell stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) or an analgesic activity (a so-called receptor antagonist).

Since the receptor agonist has all or part of the physiological activities the protein of the invention possesses, the receptor agonist is useful as a safe and low toxic pharmaceutical that has correspondingly the physiological activities. The receptor agonist is useful as a drug, e.g., an analgesic agent.

On the other hand, the receptor antagonist can suppress all or part of the physiological activities the protein of the invention possesses. Therefore, the antagonist is useful as a safe and low toxic pharmaceutical that suppresses the physiological activities.

In the case that the receptor antagonist or receptor agonist described above is used as the aforesaid pharmaceutical, the antagonist or agonist may be used orally, for example, in the form of tablets, if necessary, sugarcoated, capsules, elixirs, microcapsules etc., or parenterally in the form of injectable preparations such as a sterile solution or a suspension in water or with other pharmaceutically acceptable liquid. These preparations can be manufactured by mixing the receptor antagonist or receptor agonist described above with a physiologically acceptable carrier, flavoring agent, excipient, vehicle, antiseptic agent, stabilizer, binder, etc., in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol. The pharmaceutical compositions described above may further contain a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid injection is normally filled in an appropriate ampoule.

Since the thus obtained pharmaceutical composition is safe and low toxic, the preparation can be administered to warm-blooded animals (for example, mammals such as human, rat, mouse, guinea pig, rabbit, bird, sheep, swine, bovine, horse, cat, dog, monkey).

The dose of the receptor agonist varies depending on, for example, conditions to be treated. In oral administration, the daily dose for a normal adult (weighing 60 kg) is generally about 0.1 mg to about 100 mg, preferably about 1.0 to about 50 mg, and more preferably about 1.0 to about 20 mg of the active component. In parenteral administration, the single dose varies depending on, for example, subject to be administered, target organ, conditions, and routes for administration. For example, it is advantageous to inject intravenously to a normal adult (weighing 60 kg) the active ingredient in a daily dose of about 0.01 to about 30 mg, preferably about 0.1 to about 20 mg, and more preferably about 0.1 to about 10 mg. The corresponding dose as converted per 60 kg body weight can be administered to other animals.

The dose of the receptor antagonist varies depending on, for example, conditions to be treated. In oral administration, the daily dose for a normal adult (weighing 60 kg) is generally about 0.1 mg to about 100 mg, preferably about 1.0 to about 50 mg, and more preferably about 1.0 to about 20 mg of the active component. In parenteral administration, the single dose varies depending on, for example, subject to be administered, target organ, conditions, and routes for administration. For example, it is advantageous to inject intravenously to a normal adult (weighing 60 kg) the active ingredient in a daily dose of about 0.01 to about 30 mg, preferably about 0.1 to about 20 mg, and more preferably about 0.1 to about 10 mg. The corresponding dose as converted per 60 kg body weight can be administered to other animals.

(B) Method and kit for screening a proteinase inhibitor capable of degrading the protein of the invention

It is considered that the protein of the invention or its salt would be cleaved and inactivated by a proteinase present in vivo. Thus, by using the protein of the invention and a proteinase capable of degrading the protein of the invention, a compound that has an activity of inhibiting the proteinase capable of degrading the protein of the invention can be screened.

The compound having an activity of inhibiting the proteinase can prevent inactivation of the protein of the invention in vivo thereby to promote the activity of the protein of the invention without the intercellular contact, and can be expected to be a pharmaceutical such as an analgesic agent, etc.

That is, the present invention provides a method of screening a compound or its salt that has an activity of inhibiting a proteinase capable of degrading the protein of the invention, which comprises using the protein of the invention.

More specifically, the present invention provides:

(1) a method of screening a compound or its salt that has an activity of inhibiting a proteinase capable of degrading the protein of the invention, which comprises comparing cases (i) wherein after a proteinase capable of degrading the protein of the invention is incubated with the protein of the invention, the protein is contacted with a cell containing the receptor and (ii) wherein after a proteinase capable of degrading the protein of the invention and a test compound are incubated with the protein of the invention, the protein is contacted with a cell containing the receptor.

Specifically, the screening method of the present invention is characterized by determining and comparing the activity, for example, a receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity in the cases (i) and (ii).

More specifically, the present invention provides:

(1a) a method of screening a compound or its salt that has an activity of inhibiting a proteinase capable of degrading the protein of the invention, which comprises determining and comparing the activity, for example, a receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity, in cases (i) wherein after a proteinase capable of degrading the protein of the invention is incubated with the protein of the invention, the protein is contacted with a cell containing the receptor and (ii) wherein after a proteinase capable of degrading the protein of the invention and a test compound are incubated with the protein of the invention, the protein is contacted with a cell containing the receptor.

In the above screening method, a test compound that promotes the activity such as a cell-stimulating activity mediated by the receptor (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity, can be selected as a compound or its salt that has an activity to inhibit a proteinase capable of degrading the protein of the invention.

Human-derived or warm blooded animal-derived analgesic peptide receptors ($\delta$, $\kappa$, $\mu$) are used as the receptors for the screening methods of the present invention.

The receptors to the protein of the invention may be obtained in accordance with known methods for protein purification. The desired receptors may also be obtained by cloning DNAs encoding the receptors in accordance with known genetic engineering procedures and then following the methods of expressing the protein of the invention described above.

As the cells containing the receptor described above, which are used for the screening methods of the present invention, the same cells given as the host cells used to express the protein of the invention can be employed. Among others, CHO cells, etc. are preferred. The receptor-containing cells described above may be produced by known methods, e.g., in accordance with the methods for expressing the protein of the invention described above, etc. Cell lines such as CL8 cell line (BONE, 18, 159-169, 1996), OK cell line (AMERICAN JOURNAL OF PHYSIOLOGY, 253, E221-E227, 1987), etc. may also be used as the receptor-containing cells described above.

In the screening methods of the present invention wherein the receptor-containing cells are used, the cells may be fixed using glutaraldehyde, formalin, etc. The fixation can be effected by publicly known methods.

The same cell membrane fraction as described above may be used as the cell membrane fraction of the cell containing the receptor.

Examples of the test compound include proteins, non-proteinaceous compounds, synthetic compounds, fermentation products, cell extracts, vegetable extracts, animal tissue extracts, etc. and these compounds may be novel compounds or publicly known compounds.

In the screening methods of the present invention, the incubation of the proteinase and the protein of the invention can be carried out normally for several hours at about 37°C. Also the reaction of this incubation mixture with the cell containing the receptor can be carried out normally for several hours at about 37°C.

The receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity, can be assayed as described above.

The screening kit of the present invention comprises the protein of the invention and the proteinase capable of degrading the protein of the invention, and preferably further comprises the cell containing the receptor.

The following is an example of the screening kit of the present invention.

[Reagents for screening]

(1) Assay buffer and wash buffer

Hanks' Balanced Salt Solution (manufactured by Gibco) supplemented with 0.05% of bovine serum albumin (manufactured by Sigma). The buffers may be sterilized by filtration through a membrane filter with a 0.45 µm pore size and stored at 4°C, or may be prepared at use.

(2) Receptor preparation

CHO cells containing the receptor, etc. to the protein of the present invention are subcultured at 5 x $10^5$ cells/well on a 12-well plate at 37°C under 5% $CO_2$ and 95% air for 2 days.

(3) Preparation of the protein of the invention

The protein of the invention, its partial peptide or a salt thereof.

(4) Preparation of a proteinase capable of degrading the protein of the invention

A proteinase capable of degrading the protein of the invention.

[Procedures for assay]

(1) A proteinase capable of degrading the protein of the invention is incubated with the protein of the invention at about 37°C for several hours.
(2) A proteinase capable of degrading the protein of the invention and a test compound are incubated with the protein of the invention at about 37°C for several hours.
(3) The reaction mixtures obtained in (1) and (2) are incubated at about 37°C for several hours, respectively, with the cell containing the receptor to the protein of the invention.
(4) Next, the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity, can be assayed in accordance with the methods described above.

As above, the protein of the invention is useful as a reagent for screening the compound or its salt that has an activity of inhibiting the proteinase capable of degrading the protein of the invention.
The compound or its salt, which is obtained using the screening methods or the screening kits of the present invention, inhibits a proteinase capable of degrading the protein of the invention to suppress inactivation of the protein of the invention by the proteinase. Therefore, the compound can promote the activity such as the receptor-mediated cell-stimulating activity independent from intercellular contact (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), an analgesic activity or other activity, and is thus useful as a safe and low toxic pharmaceutical, e.g., an analgesic agent, etc.
When the compound obtained using the screening methods or screening kits of the present invention is used as a therapeutic/prophylactic agent described above, such can be implemented in a manner similar to that with the receptor agonist/antagonist described above.

(C) Method of screening a compound or its salt that promotes or inhibits intracellular signal transduction after binding of the protein of the invention to the receptor

Since the protein of the invention is capable of specifically binding to analgesic peptide receptors (δ, κ, μ) (hereinafter merely referred to as the receptors) derived from warm blooded animals (mammals such as a human, etc.), a compound or its salt that promotes or inhibits intracellular signal transduction after binding of the protein of the invention to the receptor can be screened by constructing the ligand-receptor binding assay system using the protein of the invention and the receptor.
Thus, the present invention provides a method of screening a compound or its salt that promotes or inhibits intracellular signal transduction after binding of the protein of the invention to the receptor, which comprises using the protein of the invention.

More specifically, the present invention provides:

(1) a method of screening a compound or its salt that promotes or inhibits intracellular signal transduction after binding of the protein of the invention to the receptor, which comprises comparing cases (i) wherein the protein of the invention is brought in contact with a cell containing the receptor and (ii) wherein the protein of the invention and a test compound are brought in contact with a cell containing the receptor.

Specifically, the screening method of the present invention is characterized by determining and comparing, e.g., intracellular signal transduction after binding of the protein of the invention to the receptor activity in the cases of (i) and (ii).

More specifically, the present invention provides:

(1a) a method of screening a compound or its salt that promotes or inhibits intracellular signal transduction after binding of the protein of the invention to the receptor, which comprises determining and comparing the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) in the cases of (i) wherein the protein of the invention is brought in contact with a cell containing the receptor and (ii) wherein the protein of the invention and a test compound are brought in contact with a cell containing the receptor.

In the above screening method (1a), a compound that does not inhibit the binding of the protein of the invention to the receptor but promotes the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) by the protein of the invention can be selected as a compound or its salt that promotes intracellular signal transduction after binding of the protein of the invention to the receptor. On the other hand, a compound that does not inhibit the binding of the protein of the invention to the receptor and has an action of inhibiting the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) by the protein of the invention can be selected as a compound or its salt that inhibits intracellular signal transduction after binding of the protein of the invention to the receptor.

That is, since this screening method is to screen a compound that does not affect the binding of the protein of the invention to the receptor but regulates (promotes or inhibits) intracellular signal transduction after binding of the protein to the receptor, it is desired to employ as a test compound used for the screening method a compound that is not selected as the receptor agonist/antagonist in the method of screening a receptor agonist/antagonist described above.

Analgesic peptide receptors ($\delta$, $\kappa$, $\mu$), etc. derived from warm blooded animals (mammals such as a human) are used as the receptors for the screening methods of the present invention.

The receptors to the protein of the invention may be obtained in accordance with known methods for protein purification. The desired receptors may also be obtained by cloning DNAs encoding the receptors in accordance with known genetic engineering procedures and then following the methods of expressing the protein of the invention described above.

A partial peptide obtained by suitably cleaving the full-length receptor can be employed as the partial peptide of the receptor.

The same cells given as host cells used to express the protein of the invention described above may be employed as the aforesaid cell containing the receptor, which are used for the screening methods of the present invention. Among others, CHO cells, etc. are preferred. The receptor-containing cells may be produced by known methods, e.g., in accordance with the methods for expressing the protein of the invention described above, etc. Cell lines such as CL8 cell line (BONE, 18, 159-169, 1996), OK cell line (AMERICAN JOURNAL OF PHYSIOLOGY, 253, E221-E227, 1987), etc. may also be used as the receptor-containing cells described above.

In the screening methods of the present invention in which the receptor-containing cells are used, the cells may be fixed using glutaraldehyde, formalin, etc. The fixation can be effected by publicly known methods.

The same cell membrane fraction as described above may be used as the cell membrane fraction of the receptor-containing cell described above.

Examples of the test compounds include proteins, non-proteinaceous compounds, synthetic compounds, fermentation products, cell extracts, vegetable extracts, animal tissue extracts, etc. and these compounds may be novel compounds or publicly known compounds.

In the screening methods of the present invention, the reaction of the protein of the invention with the receptor-containing cell can be carried out normally at about 37°C for several hours.

In the screening method (1a) described above, the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) by the protein of the invention can be assayed as described above.

In the above screening method (1a), when the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) by the protein of the invention is promoted by addition of a test compound, the test compound can be selected as a compound or its salt that promotes intracellular signal transduction after binding of the protein to the receptor. On the other hand, when the receptor-mediated cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) by the protein of the invention is inhibited by addition of a test compound, the test compound can be selected as a compound or its salt that promotes intracellular signal transduction after binding of the protein to the receptor.

The screening kit of the present invention comprises the protein of the invention, and preferably further comprises the receptor-containing cell.

The following is an example of the screening kit of the present invention.

[Reagents for screening]

(1) Assay buffer and wash buffer

Hanks' Balanced Salt Solution (manufactured by Gibco) supplemented with 0.05% of bovine serum albumin (manufactured by Sigma). The buffers may be sterilized by filtration through a membrane filter with a 0.45 µm pore size and stored at 4°C, or may be prepared at use.

(2) Receptor preparation

CHO cells containing the receptor to the protein of the present invention are subcultured at $5 \times 10^5$ cells/well on a 12-well plate at 37°C under 5% $CO_2$ and 95% air for 2 days.

(3) Preparation of the protein of the invention

The protein of the invention, its partial peptide or a salt thereof.

[Procedures for assay]

The cell-stimulating activity (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.) is assayed in accordance with the procedures described above.

As above, the protein of the invention is useful as a reagent for screening a compound or its salt that promotes or inhibits intracellular signal transduction after binding of the protein to the receptor.

The compound or its salt, which is obtained using the screening methods or screening kits of the invention, promotes a cell-stimulating activity mediated by the receptor after binding of the protein of the invention to the receptor (e.g., arachidonic acid release, acetylcholine release, change in intracellular $Ca^{2+}$ release, intracellular cAMP production, intracellular cGMP production, inositol phosphate production, change in cell membrane potential, phosphorylation of intracellular proteins, pH reduction, etc.), or the compound or its salt inhibits the cell-stimulating activity.

The compound or its salt that promotes intracellular signal transduction after binding of the protein of the invention to the receptor is useful as a safe and low toxic pharmaceutical, e.g., an analgesic agent, etc.

When the compound obtained using the screening methods or screening kits of the invention is employed as a therapeutic/prophylactic agent described above, such can be implemented in a manner similar to that with the receptor agonist/antagonist described above.

**<4> Antisense DNA**

An antisense DNA, which can be bound complementally to the DNA or mRNA encoding the protein of the present invention to suppress expression of the DNA, the mRNA, or the protein of the present invention, can inhibit the function of the protein of the present invention having the activity in vivo as described above, or the function of the DNA encoding the protein.
When the antisense DNA is used as the aforementioned prophylactic and/or therapeutic agent, such an agent can be used in the same way as the aforementioned prophylactic and/or therapeutic agent for various diseases comprising the protein or the DNA of the present invention.
Such an antisense DNA can also be used as a diagnostic oligonucleotide probe to investigate the existence and expression of the DNA of the present invention in tissues or cells.

**<5> Production of DNA-Introduced Animals**

The present invention provides non-human mammals having the DNA encoding the protein of the present invention (abbreviated hereinafter as "the foreign DNA of the present invention") or a modified DNA thereof (sometimes abbreviated hereinafter as "the modified foreign DNA of the present invention").
Thus, the invention provides:

(1) Non-human mammals having the foreign DNA of the present invention or the modified DNA thereof;
(2) The animals described in (1) above, wherein the non-human mammals are rodents;
(3) The animals described in (2) above, wherein the rodents are mice or rats;
(4) A recombinant vector capable of being expressed in mammals, comprising the foreign DNA of the present invention or the modified DNA thereof; and
(5) A pharmaceutical for gene therapy, comprising the recombinant vector described in (4) above.

The non-human mammals having the foreign DNA of the present invention or the modified DNA thereof (hereinafter abbreviated as "DNA-introduced animals of the present invention") can be prepared by introducing the desired foreign DNA of the present invention by a method such as the calcium phosphate method, electrical pulse method, lipofection method, agglutination method, microinjection method, particle gun method or DEAE-dextran method into germ cells and the like including unfertilized eggs, fertilized eggs, sperm and primordial cells thereof, preferably during the embryonic stage of non-human mammalian development (and more preferably during the single-cell or fertilized egg cell stage, generally before the eight-cell stage). Such DNA-introducing methods can also be used to introduce the desired foreign DNA of the present invention into somatic cells, living organs or tissue cells for cell culture or tissue culture. The DNA-introduced animals of the present invention can also be produced by fusing these cells with the aforementioned germ cells according to publicly known cell fusion methods.
Non-human mammals that can be used include for example cows, pigs, sheep, goats, rabbits, dogs, cats, guinea pigs, hamsters mice and rats. From the standpoint of preparing animal disease models, rodents are preferred which have relatively short ontogeny and life cycles and which are easy to breed, especially mice (including pure strains such as C57BL/6 and DBA2 and hybrid strains such as B6C3F1, BDF1, B6D2F1, BALB/c and ICR) and rats (such as Wistar and SD rats).
In the context of the recombinant vectors, which can be expressed in mammals, the term "mammal" applies to humans as well as non-human mammals.

The foreign DNA of the present invention refers to the DNA of the present invention that has been previously isolated and extracted from mammals, not the DNA of the present invention which the non-human mammals have intrinsically.
The modified DNA of the present invention includes a DNA in which modifications (such as mutations) have occurred in the original nucleotide sequence of the DNA of the present invention, specifically, a DNA with addition, deletion, or substitution of a nucleic acid, or an abnormal DNA.
The abnormal DNA refers to a DNA which expresses an abnormal protein of the present invention, and includes a DNA which expresses a protein which inhibits the function of the normal protein of the present invention.
The foreign DNA of the present invention may be from mammals of either the same species or different species from the target animals. When introducing the DNA of the present invention into the target animals, it is generally advantageous to use a DNA construct having the DNA ligated downstream of a promoter which can be expressed in animal cells. For example, when introducing the human DNA of the present invention, a DNA-introduced mammal can be prepared, which strongly expresses the DNA of the present invention, by microinjecting into the fertilized eggs of the target mammal, such as fertilized mouse eggs, a DNA construct (such as a vector) having the human DNA of the present invention ligated downstream of various promoters which can express a DNA derived from various mammals (such as rabbits, dogs, cats, guinea pigs, hamsters, rats or mice) having the DNA of the present invention, which is highly homologous to the human DNA.

Plasmids derived from *E. coli, B. subtilis* or yeast, bacteriophages such as λ-phage, retroviruses such as Moloney leukemia virus and animal viruses such as vaccinia virus and baculovirus may be used as the expression vector of the protein of the present invention. Among them, plasmids derived from *E. coli, B. subtilis* or yeast are preferred.

Promoters that can be used to regulate the DNA expression include promoters derived from viruses (such as simian virus, cytomegalovirus, Moloney leukemia virus, JC virus, mammary tumor virus or polio virus), and promoters derived from various mammals (humans, rabbits, dogs, cats, guinea pigs, hamsters, rats, mice, etc.) or birds (chickens, etc.), such as albumin, insulin II, uroplakin II, elastase, erythropoietin, endothelin, muscle creatine kinase, glial fibrillary acidic protein, glutathione S-transferase, platelet derived growth factor β, keratin K1, K10 and K14, collagen Type I and Type II, cyclic AMP-dependent protein kinase βI subunit, dystrophin, tartaric acid-resistant alkali phosphatase, cardiac sodium diuretic factor, endothelial receptor tyrosine kinase (normally abbreviated as Tie2), sodium-potassium ATPase (Na,K-ATPase), neurofilament light chain, metallothionein I and IIA, tissue inhibitor of metalloproteinase-1, MHC class I antigen (H-2L), H-ras, renin, dopamine β-hydroxylase, thyroid peroxidase (TPO), polypeptide chain elongation factor 1α (EF-1α), β-actin, α- and β-myosin heavy chains, myosin light chains 1 and 2, myelin basic protein, thyroglobulin, Thy-1, immunoglobulin, H chain variable region (VNP), serum amyloid P component, myoglobin, troponin C, smooth muscle α-actin, preproenkephalin A, vasopressin and other promoters. Particularly suitable are cytomegalovirus promoter, human polypeptide chain elongation factor 1α (EF-1α) promoter and human and chicken β-actin promoters, which allow strong expression throughout the body.

The aforementioned vectors should preferably have the sequence (generally called the terminator) which terminates transcription of the target mRNA in DNA-introduced mammals. Each terminator DNA sequence derived from viruses, mammals and birds can be used, and the simian virus SV40 terminator is used preferably.

In order to achieve greater expression of the desired foreign DNA, it is also possible depending on the purpose to attach various DNA splicing signals, enhancer regions or parts of eukaryote-derived DNA introns either 5' upstream from the promoter region, between the promoter region and the translation region or 3' downstream from the translation region.

Translation regions for the normal protein of the present invention may be obtained as all or part of DNA derived from the livers, kidneys, thyroid glands or fibroblast cells of various mammals (such as humans, rabbits, dogs, cats, guinea pigs, hamsters, rats, mice, etc.) or genome DNA from various commercial genomic DNA libraries, or may be obtained from a stock of complement DNA prepared by publicly known methods from mRNA derived from livers, kidneys, thyroid glands or fibroblast cells. In the case of abnormal foreign DNA, mutated translation regions can be prepared by point mutagenesis of the translation regions of normal proteins obtained from the aforementioned cells or tissue.

Such translation regions can be prepared as DNA constructs capable of expression in DNA-introduced animals by conventional genetic engineering methods of attachment downstream from the aforementioned promoter, or if desired, upstream from the terminator site.

Introduction of the DNA of the present invention at the fertilized egg cell stage ensures that the DNA of the present invention will be present in all germ and somatic cells of the target mammal. The presence of the DNA of the present invention in the animal's germ cells after DNA introduction means that all the animal's progeny will retain the DNA of the present invention in all their germ and somatic cells.

The offspring of animals of this species that inherit DNAs will have the DNA of the present invention in all their germ and somatic cells.

Non-human mammals in which the normal foreign DNA of the present invention has been introduced can be bred to confirm stable retention of the DNA, and can be successively reared in a normal environment as a animal having such a DNA. Introduction of the DNA of the present invention at the fertilized egg cell stage ensures that the DNA of the present invention will be present in excess in all germ and somatic cells of the target mammal. The excessive presence of the DNA of the present invention in the animal's germ cells after DNA introduction means that all the animal's progeny will retain an excess of the DNA of the present invention in all their germ and somatic cells. The offspring of animals of this species that inherit DNAs will have an excess of the DNA of the present invention in all their germ and somatic cells. It is possible to obtain homozygotic animals with the introduced DNA in both homologous chromosomes, and breed the male and female so that all the progeny have the DNA in excess.

The normal DNA of the present invention is strongly expressed in non-human mammals having normal DNA of the present invention, and promotion of the function of the intrinsic normal DNA results ultimately in hyperfunction of the protein of the present invention, making these animals useful as a disease model. For example, the normal DNA-introduced animals of the present invention can be used to elucidate the pathology of hyperfunction of the protein of the present invention and other diseases related to the protein of the present invention, and to investigate therapies for these conditions.

Furthermore, since mammals in which the normal foreign DNA of the present invention has been introduced have symptoms of increased free protein of the present invention, it can also be used in screening tests for pharmaceuticals for treatment of conditions related to the protein of the present invention.

Non-human mammals having the abnormal foreign DNA of the present invention can be bred to confirm stable retention of the DNA, and can be successively reared in a normal environment as animals having such a DNA. Furthermore, the desired DNA can be incorporated into one of the aforementioned plasmids and used as a raw material. A DNA construct with a promoter can be produced according to ordinary genetic engineering techniques. Introduction of the abnormal DNA of the present invention at the fertilized egg stage ensures that the abnormal DNA of the present invention is present in all the germ and somatic cells of the target mammal. The presence of the abnormal DNA of the present invention in the animal's germ cells after DNA introduction means that all the animal's progeny will retain the abnormal DNA of the present invention in all their germ and somatic cells. The offspring of animals of this species that inherit DNAs will have the abnormal DNA of the present invention in all their germ and somatic cells. It is possible to obtain homozygote animals with the introduced DNA in both homologous chromosomes, and breed the male and female so that all the progeny have this DNA.

The abnormal DNA of the present invention is strongly expressed in non-human mammals having the abnormal DNA of the present invention, and inhibition of the function of the intrinsic normal DNA can ultimately result in inactive dysfunction of the protein of the present invention, making these animals useful as a disease model. For example, it is possible to use the abnormal DNA-introduced animals to elucidate the pathology of inactive dysfunction of the protein of the present invention, and to investigate therapies for this condition.

In terms of specific possible applications, an animal that strongly expressed abnormal DNA of the present invention could be a model for elucidating inhibition of normal protein function (dominant negative effect) caused by the abnormal protein of the present invention in inactive dysfunction of the protein of the present invention.

Moreover, since the mammals introduced with the abnormal foreign DNA of the present invention have symptoms of increased free protein of the present invention, they can be used in screening tests for pharmaceuticals for treatment of inactive dysfunction of the protein of the present invention.

Other applicability for the aforementioned two types of DNA-introduced animals of the present invention include:

(1) use as cell sources for tissue culture;
(2) direct analysis of DNA or RNA in the tissue of DNA-introduced mammals of the present invention or analysis of proteins in tissues to elucidate connections with proteins that are specifically expressed or activated by the protein of the present invention;
(3) researching the function of cells derived from a tissue which is generally difficult to culture, by using cells derived from a tissue having the DNA of the present invention, wherein such cells can be cultured by standard tissue culture techniques;
(4) screening for pharmaceuticals that enhance the cellular functions using the cells described in (3) above; and
(5) isolation and purification of the modified protein of the present invention, and production of antibodies thereto.

The DNA-introduced animals of the present invention could also be used to investigate the clinical symptoms of diseases related to the protein of the present invention, including inactive dysfunction of the protein of the present invention, to obtain more detailed pathological findings from various organs of a disease model related to the protein of the present invention, to develop new therapies, and to contribute to research and therapies for secondary conditions stemming from such diseases.

It is also possible to remove various organs from the DNA-introduced animals of the present invention, cut them up, use a protease such as trypsin to obtain free DNA-introduced cells, and prepare a cell line from the culture or cultured cells. By allowing cells producing the protein of the present invention to be specified and their signal transduction mechanism to be investigated to find abnormalities therein, these animals also provide an effective research tool for understanding the protein of the present invention and action thereof.

Moreover, the DNA-introduced animals of the present invention might also be used to provide a rapid method of screening pharmaceuticals in the development of treatments for of diseases related to the protein of the present invention, including inactive dysfunction of the protein of the present invention, using the testing and assay methods described above. The DNA-introduced animals of the present invention or vectors expressing foreign DNA of the present invention could also be used to investigate and develop DNA therapies for diseases related to the protein of the present invention.

**<6> Production of Knockout Animals**

The present invention provides non-human mammal's embryonic stem cells in which the DNA of the present invention is inactivated; and non-human mammals which fail to express the DNA of the present invention.

Specifically, there are provided:

(i) Non-human mammal's embryonic stem cells in which the DNA of the present invention is inactivated;
(ii) The embryonic stem cells described in (i) above, which have the *E. coli* β-galactosidase gene as a reporter gene;
(iii) The embryonic stem cells described in (i) above, which are neomycin resistant;
(iv) The embryonic stem cells described in (i) above, wherein the non-human mammal is a rodent;
(v) The embryonic stem cells described in (iv) above, wherein the rodent is a mouse;
(vi) A non-human mammal, which fails to express the DNA of the present invention;
(vii) The mammal described in (vi) above, in which a reporter gene can be expressed under the control of a promoter for the DNA of the present invention;
(viii) The mammal described in (vii) above, wherein the reporter gene is the *E. coli* β-galactosidase gene;
(ix) The mammal described in (vi) above, wherein the non-human mammal is a rodent;
(x) The non-human mammal described in (ix) above, wherein the rodent is a mouse; and
(xi) A screening method for a compound or a salt thereof which enhances the promoter activity of the DNA of the present invention, wherein a test compound is administered to the animal described in (vii) above, and expression of the reporter gene is detected.

Non-human mammal embryonic stem cells in which the DNA of the present invention is inactivated are the embryonic stem cells (abbreviated hereinafter as "ES cells") of non-human mammals wherein either DNA expression ability is suppressed by the addition of an artificial modification to the DNA of the present invention in the non-human mammal, or wherein the activity of the protein of the present invention encoded by said DNA has effectively been eliminated so that the DNA is effectively incapable of expressing the protein of the present invention (sometimes referred to hereinafter as the knockout DNA of the present invention). The non-human mammals to be used are as described above.

Methods for artificially modifying the DNA of the present invention include for example using genetic engineering techniques to delete all or part of the DNA sequence, or to insert or substitute other DNA. The knockout DNA of the present invention is produced when these modification result in displacement of the codon reading frame or disruption of the function of the promoter or exon.

Specific examples of non-human mammal embryonic stem cells in which the DNA of the present invention is inactivated (abbreviated hereinafter as ES cells of the present invention comprising inactivated DNA or knockout ES cells of the present invention) include those in which the DNA of the present invention in the target non-human mammal is isolated, a drug-resistant gene of which typical examples are neomycin-resistant, hygromycin-resistant or other drug-resistant genes, or a reporter gene or the like of which typical examples are lacZ (β-galactosidase gene) or cat (chloramphenicol acetyltransferase gene), is inserted into the exon to disrupt the function of the exon, or else a DNA sequence (such as polyA addition signal) which terminates gene transcription is inserted into the intron between the exons to completely prevent mRNA synthesis, and ultimately a DNA chain having a DNA sequence constructed to disrupt genes (abbreviated hereinafter as "the targeting vector") is introduced into the chromosomes of the animal by homologous recombination, for example. The knockout ES cells of the present invention can be selected by analyzing the resulting ES cells either by the southern hybridization method using a DNA sequence on or near the DNA of the present invention as a probe, or by the PCR method using as primers a DNA sequence on the targeting vector and a DNA sequence of a nearby region other than the DNA of the present invention used in creating the targeting vector.

For the original ES cells used to inactivate the DNA of the present invention by homologous recombination or the like, it is possible to use already established cells as described above, or to establish new ones according to the publicly known Evans and Kaufman methods. For example, 129 mouse ES cell lines are currently in general use, but since the immunological background is unclear, a good way to establish a pure line for which the immunological and genetic background is known would be to use C57BL/6 mice or else BDF1 mice (F1 of C57BL/6 and DBA/2) in which the low ovum collection of C57BL/6 mice has been improved by cross-breeding with DBA/2 mice. Not only do BDF1 mice have high ovum collection and sturdy ova, but since they are based on C57BL/6 mice, the genetic background of ES cells obtained therefrom can be restored by back crossing with C57BL/6 mice when preparing a disease model mouse.

The blastocyst 3.5 days after fertilization is generally used in establishing ES cells, but many early embryos can be obtained efficiently by collecting 8-cell embryos and culturing them to the blastocyst stage.

Either female or male ES cells can be used, but generally male ES cells are more useful for preparing reproductive lineage chimeras. Females and males should be distinguished as quickly as possible to reduce the work of complex cultures.

One method of distinguishing female and male ES cells is to use PCR to amplify and detect the genes of the sex-determining region of the Y chromosome. Previously, about $10^6$ cells were required for karyotype analysis, but this method uses only about one colony's worth of ES cells (about 50 cells), allowing primary selection of ES cells by sexing the cells at the initial culture stage. This greatly reduces the work of the initial culture stage by allowing early selection of male cells.

Secondary selection can be accomplished for example by using the G-banding method to confirm the number of chromosomes. Ideally, 100% of the ES cells should have a normal number of chromosomes, but when this is difficult due to the physical manipulation used in establishing the cells, for example. ES cell should be cloned again into the normal cells (for example, those having the normal mouse chromosome number of 2n = 40) after the ES cell genes are knocked out.

The resulting embryonic stem cell line will normally be highly productive, but since it can easily lose the power of ontogenesis, successive cultures must be performed very carefully. For example, culture can be performed in a carbon dioxide incubator (preferably with 5% carbon dioxide, 95% air or 5% oxygen, 5% carbon dioxide, 90% air) at about 37°C in the presence of LIF (1-10000 U/ml) on suitable feeder cells such as STO fibroblasts. During passage, treatment with a trypsin/EDTA solution (normally 0.001-0.5% trypsin/0.1-5 mM EDTA, preferably about 0.1% trypsin/l mM EDTA) is used to produce a single cell, which is seeded on a previously prepared feeder cell. Such a passage is normally performed every 1-3 days, and at that time if a cell is found to be morphological abnormal the cultured cells are discarded.

ES cells can be differentiated into a variety of cells types, such as musculus longus capitis, visceral muscle or cardiac muscle cells, by culturing under suitable conditions either in a monolayer culture until they reach high density, or else in a float culture until they form a cell clump (M.J. Evans and M. H. Kaufman, *Nature, Vol*. 292, 154 (1981); G. R. Martin, *Proc. Natl. Acad. Sci. USA*, Vol. 78, 7634 (1981); T. C. Doetschman et al, *Journal of Embryology and Experimental Morphology,* Vol. 87, 27 (1985). Cells obtained by differentiation of the ES cells of the present invention that fail to express the DNA of the present invention are useful in investigating *in vitro* cellular functions of the protein of the present invention.

Non-human mammals, which do not express the DNA of the present invention can be distinguished from normal animals by measuring the amount of mRNA by publicly known methods and indirectly comparing the amount expressed. The non-human mammals to be used are as described above.

In the non-human mammals which do not express the DNA of the present invention, the DNA of the present invention can be knocked out for example by introducing a targeting vector created as described above into mouse embryonic stem cells or mouse egg cells, resulting in the replacement, by genetic homologous recombination, of the DNA of the present invention on the chromosomes of the mouse's embryonic stem cells or egg cells with a DNA sequence in the targeting vector in which the DNA of the present invention is inactivated.

Cells in which the DNA of the present invention is knocked out can be evaluated either by the southern hybridization method using a DNA sequence on or near the DNA of the present invention as a probe, or by the PCR method using as primers a DNA sequence on the targeting vector and the DNA sequence of a nearby region other than the mouse-derived DNA of the present invention used in creating the targeting vector. When using non-human mammal embryonic stem cells, a cell line in which the DNA of the present invention is inactivated can be cloned by homologous recombination, and the cells injected into the embryos or blastocysts of a non-human mammal at a suitable stage such as the 8-cell stage. The resulting chimera embryo is then transplanted to the uterus of the non-human mammal, which has been made falsely pregnant. The resulting animal is a chimera animal comprising both cells with the normal DNA locus of the present invention and the artificially modified DNA locus of the present invention.

If some of the reproductive cells of this chimera animal have modified DNA of the present invention, individuals all of whose tissues are made up of cells with the artificial modification included in the DNA locus of the present invention can be selected by evaluation of coat color, for example, from a population produced by the breeding of this chimera with a normal individual. The individuals obtained in this way normally are hetero for failure to express the protein of the present invention, and by breeding such animals it is possible to obtain from their offspring individuals that are homo for failure to express the protein of the present invention.

When using egg cells, it is possible to obtain transgenic non-human mammals with the targeting vector inserted into their chromosomes by injecting a DNA solution into the egg cell nucleus with microinjection. These transgenic non-human mammals are obtained by selection of those having mutations to the genetic locus of the present invention with homologous recombination.

Individuals in which the DNA of the present invention has been knocked out in this way can be successively reared in a normal environment after confirmation that the DNA is knocked out in animals obtained by breeding.

Reproductive lineages can also be obtained and maintained by ordinary methods. Namely, female and male animals having the inactivated DNA can be bred to obtain homozygous animals with the inactivated DNA in both homologous chromosomes. The resulting homozygote animals can be efficiently obtained by rearing so that for each mother animal there is one normal individual and multiple homozygote individuals. By breeding female and male heterozygous animals, homozygous and heterozygous animals with the inactivated DNA are successively produced.

Non-human mammal embryonic stem cells in which the DNA of the present invention is inactivated are extremely useful in preparing non-human mammals which fail to express the DNA of the present invention. Moreover, because a mouse which fails to express the protein of the present invention lack various kinds of biological activity which may be induced by the protein of the present invention, it can be a model for various diseases stemming from inactivation of the biological activity of the protein of the present invention, and is therefore useful in finding the causes and investigating therapeutic methods for such diseases.

In an animal which fails to express the protein of the present invention through replacement of the structure gene of the protein of the present invention with a reporter gene, since the reporter gene is under the control of a promoter for the protein of the present invention, the activity of the promoter for the protein of the present invention can be detected by tracing expression of the substance encoded by the reporter gene. For example, when a part of the DNA region encoding the protein of the present invention is replaced by an *E. coli*-derived β-galactosidase gene (lacZ), β-galactosidase is expressed instead of the protein of the present invention in a tissue which would usually express the protein of the present invention. Consequently, staining with a reagent such as 5-bromo-4-chloro-3-indolyl-β-galactopyranoside (X-gal) which is a substrate for β-galactosidase is a simple way of observing the expression of the protein of the present invention in a living body of animal. Specifically, a mouse lacking the protein of the present invention or a tissue section thereof is fixed with glutaraldehyde or the like and washed with Dulbecco's phosphate buffered saline (PBS), then reacted for about 30 minutes to an hour at room temperature or about 7°C in a staining solution containing X-gal. The tissue samples are then washed in a 1 mM EDTA/PBS solution to stop the β-galactosidase reaction, and the resulting color reaction is observed. The mRNA which encodes lacZ may also be detected by ordinary methods.

Thus, non-human animals which fail to express the protein of the present invention are extremely useful for screening compounds or salts thereof which enhance or inhibit the activity of promoter for the protein of the present invention, and could contribute greatly to discovering the causes of various diseases stemming from failure to express the protein of the present invention, and to the development of pharmaceuticals for treatment of such diseases.

In the specification and drawings, abbreviations of bases and amino acids are based on the abbreviations of the IUPAC-IUB Commission on Biochemical Nomenclature or the conventional abbreviations used in the art, examples of which are shown below. An amino acid that has an optical isomer takes its L form unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| RNA | : ribonucleic acid |
| mRNA | : messenger ribonucleic acid |
| dATP | : deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |
| EDTA | : ethylenediaminetetraacetic acid |
| SDS | sodium dodecyl sulfate |
| Gly | : glycine |
| Ala | : alanine |
| Val | : valine |
| Leu | : leucine |
| Ile | : isoleucine |
| Ser | : serine |
| Thr | : threonine |
| Cys | : cysteine |
| Met | : methionine |
| Glu | glutamic acid |
| Asp | : aspartic acid |
| Lys | : lysine |
| Arg | : arginine |
| His | : histidine |
| Phe | : phenylalanine |
| Tyr | : tyrosine |

| | |
|---|---|
| Trp | : tryptophan |
| Pro | : proline |
| Asn | : asparagine |
| Gln | : glutamine |
| pGlu | : pyroglutamic acid |
| Me | : methyl |
| Et | : ethyl |
| Bu | : butyl |
| Ph | : phenyl |
| TC | : thiazolidine-4(R)-carboxamide |

The substituents, protective groups and reagents, which are frequently used throughout the specification, are shown by the following abbreviations.

| | |
|---|---|
| Tos | : p-toluenesulfonyl |
| CHO | : formyl |
| Bzl | : benzyl |
| $Cl_2$-Bzl | : 2,6-dichlorobenzyl |
| Bom | : benzyloxymethyl |
| Z | : benzyloxycarbonyl |
| Cl-Z | : 2-chlorobenzyloxycarbonyl |
| Br-Z | : 2-bromobenzyloxycarbonyl |
| Boc | : t-butoxycarbonyl |
| DNP | : dinitrophenol |
| Trt | : trityl |
| Bum | : t-butoxymethyl |
| Fmoc | : N-9-fluorenylmethoxycarbonyl |
| HOBt | : 1-hydroxybenztriazole |
| HOOBt | : 3,4-dihydro-3-hydroxy-4-oxo-1,2,3-benzotriazine |
| HONB | : 1-hydroxy-5-norbornene-2,3-dicarboximide |
| DCC | : N,N'-dicyclohexylcarbodiimide |

The SEQ ID NOs (sequence identification number) in the Sequence Listing of the present specification indicate the following sequences, respectively.

[SEQ ID NO: 1]

This shows the amino acid sequence of the human-derived protein of the present invention.

[SEQ ID NO: 2]

This shows the nucleic acid sequence of the DNA encoding the human-derived protein of the present invention.

[SEQ ID NO: 3]

This shows the nucleic acid sequence of a primer OPF used in Example 1.

[SEQ ID NO: 4]

This shows the nucleic acid sequence of a primer OPR used in Example 1.

[SEQ ID NO: 5]

This shows the nucleic acid sequence disclosed under the deposit number AC002107 in GenBank.
A transformant *Escherichia coli* JM109/pTA0PI5 obtained in Example 1 is on deposit with Institute for Fermentation (IFO)(2-17-85, Juso Honcho, Yodogawa-ku, Osaka-shi, Osaka, Japan) under the Accession Number IFO 16451 since July 4, 2000, and with International Patent Organism Depositary, National Institute of Advanced Industrial Science and Technology (National Institute of Bioscience and Human Technology (NIBH), Agency of Industrial Science and Technology, the Ministry of International Trade and Industry)(1-1-3, Higashi, Tsukuba-shi, Ibaraki, Japan) under the Accession Number FERM BP-7229 since July 17, 2000.

## EXAMPLES

The present invention is described in more detail with reference to the following examples, being not intended to limit the scope of the present invention thereto. The genetic procedures using Escherichia coli were performed according to methods described in the "Molecular Cloning".

**Example 1:** Cloning of cDNA of a novel analgesic peptide precursor from human testis cDNA fraction by the PCR method

The amplification by PCR was performed using human testis cDNA (purchased from Clonetech, Inc.) as a template, and the following two different synthetic DNA:

OPF: 5'-CTCAGTGGCTGAAGGCACATGCGTCAC-3' (SEQ ID NO: 3)
OPR: 5'-GGGAGGCTTAGGCTTGGCTCATTTGAA-3' (SEQ ID NO: 4).

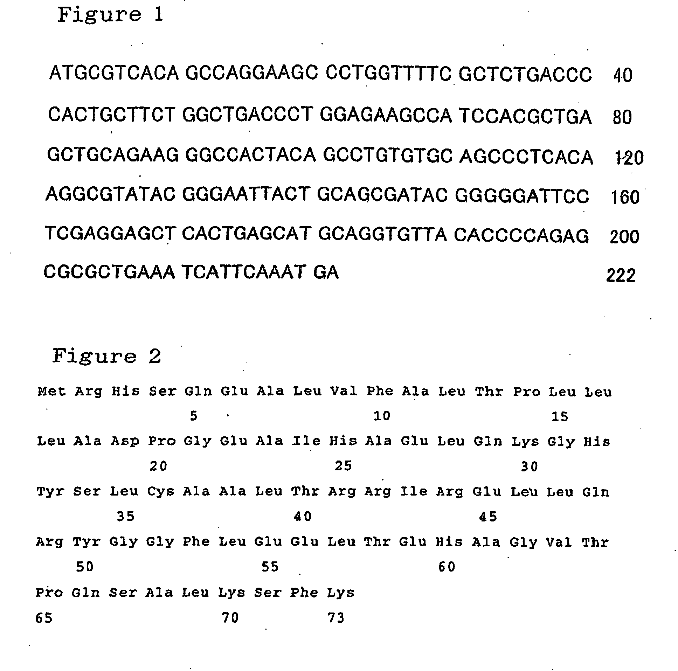

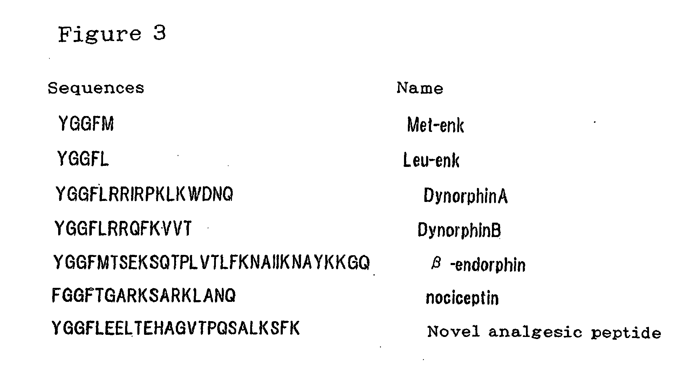

The reaction solution for PCR was a total volume of 25 µl composed of 2 µl cDNA solution, 0.5 µl OPF (10 µM), 0.5 µl OPR (10 µM), 2.5 µl 10x buffer solution attached, 2.5 µl dNTP (10 mM), 0.5 µl Klen Taq (Clonetech), and 16.5 µl distilled water. The reaction solution was subjected to PCR on Thermal Cycler 9600. The PCR conditions were denaturation at 95 °C for 2 minutes, and then 38 cycles of 98 °C for 10 seconds, 65 °C for 20 seconds, and 72 °C for 20 seconds. After a PCR product of about 250 bp was detected by subjecting an aliquot of the resulting solution to electrophoresis, the PCR product was purified using Quiagen PCR Purification Kit, and the sequence thereof was directly determined to give the sequence shown in Fig. 1 (SEQ ID NO: 2). Fig. 2 shows the amino acid sequence (SEQ ID NO: 1) predicted from the DNA sequence shown in Fig. 1. By comparing the predicted amino acid sequence with other known sequences, it is found that the predicted amino acid sequence and an analgesic peptide derived from pituitary have the highly conserved amino acid sequence Tyr-Gly-Gly-Phe-Leu (Fig. 3). The DNA thus obtained was inserted into the vector pCR2 using TA Cloning Kit (Invitrogen), and then introduced into *E.coli* JM109 to give a transformant *E.coli* JM109/pTA0PI5.

## INDUSTRIAL APPLICABILITY

The protein of the present invention and the DNA encoding the same are useful as pharmaceuticals such as an analgesic agent. In addition, the protein of the present invention and the DNA encoding the same are useful as reagents for screening a compound capable of enhancing or inhibiting the activity of the protein of the present invention. Further, antibodies to the protein of the present invention are able to recognize specifically to the protein of the present invention, and thus can be used for detection, quantification, neutralization, and the like, of the protein of the present invention contained in a test liquid.

## Claims

1. A protein or a salt thereof comprising the same or substantially the same amino acid sequence as that shown by SEQ ID NO: 1.

2. A partial peptide of the protein described in claim 1, an amide, an ester or a salt thereof.

3. A DNA comprising a DNA having a nucleic acid sequence encoding the protein described in claim 1 or the partial peptide described in claim 2, excluding the DNA having the nucleic acid sequence shown by SEQ ID NO: 5.

4. The DNA described in claim 3 having the nucleic acid sequence shown by SEQ ID NO: 2.

5. A recombinant vector comprising the DNA described in claim 4.

6. A transformant, which is transformed with the recombinant vector described in claim 5.

7. A method of producing the protein or a salt thereof described in claim 1 or the partial peptide, an amide, an ester or a salt thereof described in claim 2, which comprises culturing the transformant described in claim 6 to produce and accumulate the protein described in claim 1 or the partial peptide described in claim 2; and recovering it.

8. A pharmaceutical composition comprising the protein or a salt thereof described in claim 1, or the partial peptide, an amide, an ester or a salt thereof described in claim 2.

9. A pharmaceutical composition comprising the DNA described in claim 3.

**10.** The pharmaceutical composition described in claim 8 or 9, which has the analgesic action.

**11.** An antibody to the protein or a salt thereof described in claim 1, or the partial peptide, an amide, an ester or a salt thereof described in claim 2.

**12.** A method of screening a receptor agonist or antagonist, which comprises using the protein or a salt thereof described in claim 1, or the partial peptide, an amide, an ester or a salt thereof described in claim 2.

**13.** A kit for screening a receptor agonist or antagonist, which comprises the protein or a salt thereof described in claim 1, or the partial peptide, an amide, an ester or a salt thereof described in claim 2.

**14.** The receptor agonist or antagonist, which is obtained using the screening method described in claim 12 or the screening kit described in claim 13.

**15.** A pharmaceutical composition comprising the receptor agonist or antagonist described in claim 14.

**16.** The pharmaceutical composition described in claim 15, which has the analgesic action.

**17.** A method of relieving pain in a mammal, which comprises administering to a mammal an effective amount of the receptor agonist described in claim 14.

**18.** Use of the receptor agonist described in claim 14 for producing an analgesic agent.

**19.** A method of relieving pain in a mammal, which comprises administering to a mammal an effective amount of the protein or a salt thereof described in claim 1, or the partial peptide, an amide, an ester or a salt thereof described in claim 2.

**20.** Use of the protein or a salt thereof described in claim 1, or the partial peptide, an amide, an ester or a salt thereof described in claim 2 for producing an analgesic agent.

**21.** A method of relieving pain in a mammal, which comprises administering to a mammal an effective amount of the DNA described in claim 3.

**22.** Use of the DNA described in claim 3 for producing an analgesic agent.

## Figure 1

```
ATGCGTCACA GCCAGGAAGC CCTGGTTTTC GCTCTGACCC   40
CACTGCTTCT GGCTGACCCT GGAGAAGCCA TCCACGCTGA   80
GCTGCAGAAG GGCCACTACA GCCTGTGTGC AGCCCTCACA  120
AGGCGTATAC GGGAATTACT GCAGCGATAC GGGGGATTCC  160
TCGAGGAGCT CACTGAGCAT GCAGGTGTTA CACCCCAGAG  200
CGCGCTGAAA TCATTCAAAT GA                     222
```

## Figure 2

```
Met Arg His Ser Gln Glu Ala Leu Val Phe Ala Leu Thr Pro Leu Leu
                5                   10                  15
Leu Ala Asp Pro Gly Glu Ala Ile His Ala Glu Leu Gln Lys Gly His
            20                  25                  30
Tyr Ser Leu Cys Ala Ala Leu Thr Arg Arg Ile Arg Glu Leu Leu Gln
        35                  40                  45
Arg Tyr Gly Gly Phe Leu Glu Glu Leu Thr Glu His Ala Gly Val Thr
    50                  55                  60
Pro Gln Ser Ala Leu Lys Ser Phe Lys
65                  70          73
```

Figure 3

| Sequences | Name |
|---|---|
| YGGFM | Met-enk |
| YGGFL | Leu-enk |
| YGGFLRRIRPKLKWDNQ | DynorphinA |
| YGGFLRRQFKVVT | DynorphinB |
| YGGFMTSEKSQTPLVTLFKNAIIKNAYKKGQ | β -endorphin |
| FGGFTGARKSARKLANQ | nociceptin |
| YGGFLEELTEHAGVTPQSALKSFK | Novel analgesic peptide |

## INTERNATIONAL SEARCH REPORT

International application No.
PCT/JP01/05970

A. CLASSIFICATION OF SUBJECT MATTER
Int.Cl[7] C12N15/12, C12P21/02, C07K14/47, C07K16/18, C12N1/15, C12N1/19, C12N1/21, C12N5/00, A61K38/17, A61K31/7088, G01N33/68, A61P25/04

According to International Patent Classification (IPC) or to both national classification and IPC

B. FIELDS SEARCHED

Minimum documentation searched (classification system followed by classification symbols)
Int.Cl[7] C12N15/12, C12P21/02, C07K14/47, C07K16/18, C12N1/15, C12N1/19, C12N1/21, C12N5/00, A61K38/17, A61K31/7088, G01N33/68, A61P25/04

Documentation searched other than minimum documentation to the extent that such documents are included in the fields searched

Electronic data base consulted during the international search (name of data base and, where practicable, search terms used)
SwissProt/PIR/GeneSeq, Genbank/EMBL/DDBJ/GeneSeq, MEDLINE, BIOSIS (DIALOG)

C. DOCUMENTS CONSIDERED TO BE RELEVANT

| Category* | Citation of document, with indication, where appropriate, of the relevant passages | Relevant to claim No. |
|---|---|---|
| A | Madeleine Cochet et al., "Characterization of the structural gene and putative 5'-regulatory sequences for human proopiomelanocortin", Nature, (1982), Vol.297, pages 335 to 339 | 1-13,20,22 |
| A | Masaharu NODA et al., "Isolation and structural organization of the human preproenkephalin gene", Nature, (1982), Vol.297, pages 431 to 434 | 1-13,20,22 |
| A | Saburo HORIKAWA et al., "Isolation and structural organization of the human preproenkephalin B gene", Nature, (1983), Vol.306, pages 611 to 614 | 1-13,20,22 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 September, 2001 (19.09.01) | 09 October, 2001 (09.10.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/05970

**Box I Observations where certain claims were found unsearchable (Continuation of item 1 of first sheet)**

This international search report has not been established in respect of certain claims under Article 17(2)(a) for the following reasons:

1. ☒ Claims Nos.: 17,19,21
because they relate to subject matter not required to be searched by this Authority, namely:

The inventions as set forth in these claims pertain to "analgesic methods" which are recognized as methods for treatment wherein a substance having a pharmacological effect is administered to mammals including humans. Thus, the inventions as set forth in these claims pertain to methods for treatment of the human body by therapy.

2. ☒ Claims Nos.: 14-18
because they relate to parts of the international application that do not comply with the prescribed requirements to such an extent that no meaningful international search can be carried out, specifically:

Although the statement in page 42, lines 10 to 29 of the description is taken into consideration concerning the "agonist" and the "antagonist" described in the above claims, it is completely unknown what particular compounds are involved in the scopes of the "agonist" and the "antagonist" and what are not. Namely, the above claims are described in an extremely unclear manner. Such being the case, no meaningful international search can be practices on these claims.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)